(19) Europäisches Patentamt
European Patent Office
Office européen des brevets

(11) EP 4 248 951 A1

(12) **EUROPEAN PATENT APPLICATION**
published in accordance with Art. 153(4) EPC

(43) Date of publication:
**27.09.2023 Bulletin 2023/39**

(21) Application number: **21910741.4**

(22) Date of filing: **21.12.2021**

(51) International Patent Classification (IPC):
***A61K 9/19*** (2006.01) ***A61K 38/13*** (2006.01)
***F26B 5/06*** (2006.01)

(52) Cooperative Patent Classification (CPC):
**A61K 9/19; A61K 38/13; F26B 5/06**

(86) International application number:
**PCT/JP2021/047164**

(87) International publication number:
**WO 2022/138598 (30.06.2022 Gazette 2022/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.12.2020 JP 2020211922**

(71) Applicant: **CHUGAI SEIYAKU KABUSHIKI KAISHA**
**Tokyo 115-8543 (JP)**

(72) Inventors:
- **GOTO, Atsumi**
  **Tokyo 115-8543 (JP)**
- **ENOMOTO, Taro**
  **Tokyo 115-8543 (JP)**

(74) Representative: **Vossius & Partner Patentanwälte Rechtsanwälte mbB**
**Siebertstraße 3**
**81675 München (DE)**

# (54) MEDICINE PRODUCTION METHOD INCLUDING STEP OF FREEZE-DRYING WITH MIXED SOLVENT

(57) An object of the present invention is to provide a medicine production method including a freeze-drying method that can reduce the residual solvent concentration in a wide substrate concentration range from low concentration to high concentration. The medicine production method according to the present invention includes the following steps:
(1) providing a mixed solution containing water, an organic solvent, and a substance to be freeze-dried; and
(2) subjecting the mixed solution to freeze-drying,
wherein a weight percentage of the organic solvent based on a total weight of water and the organic solvent in the step (1) is 68% by weight or more and 99% by weight or less.

EP 4 248 951 A1

## Description

### Technical Field

The present invention relates to a medicine production method including a freeze-drying step with a mixed solvent.

### Background Art

A freeze-drying method is an effective means not only when a non-crystalline compound is taken out, but also in the handling of an unstable compound or the design of an injection preparation (Non Patent Literatures 1 and 2). Above all, freeze-drying of an aqueous solution is a well-known method. However, freeze-drying of an aqueous solution requires a large production cost because the sublimation rate of ice is low and takes time (Non Patent Literature 2). In addition, freeze-drying using a mixed solvent system of an organic solvent and water is paid attention. Since some organic solvents have a higher sublimation pressure than water and a lower latent heat of sublimation than water, it is expected to shorten the drying time along with the improvement in sublimation rate, and further, improve the stability of a bulk solution and a dried material when a compound unstable in water is targeted (Non Patent Literature 3). For example, the cases where solvents such as t-butanol, dimethyl sulfoxide, 1,4-dioxane, and acetonitrile are used as the organic solvent are reported (Non Patent Literatures 3, 4, 5, and 6). Among them, the most widely evaluated organic solvent/water mixed solvent system is the t-butanol/water mixed system (Non Patent Literature 3). The t-butanol/water mixed system can be completely frozen by a commercial freeze drier, and t-butanol has a higher vapor pressure and a lower latent heat of sublimation as compared with water, and is thus easily sublimated during primary drying, so that the improvement of the sublimation rate is enabled. In addition, t-butanol has a relatively low toxicity as compared with other organic solvents.

On the other hand, control of the concentration of a residual solvent having toxicity is an important problem in freeze-drying using a mixed solvent of an organic solvent and water, t-Butanol (TBA), one of the solvents used in freeze-drying, is classified as a class 2 solvent in the ICH Q3C guideline and is a solvent whose residual solvent concentration in medicine should be regulated (Non Patent Literature 7).

Here, it is known that, in freeze-drying, the higher the concentration of a substrate to be freeze-dried is, the more difficult the control of the residual solvent concentration is (Non Patent Literature 4). For example, Non Patent Literature 8 reports that, when a compound to be freeze-dried is not uniformly dissolved in a solvent, distillation of TBA is difficult. That is, when freeze-drying is carried out using TBA-water, the mixed solution is separated into a layer rich in the substrate and a layer rich in TBA. The layer separation of the solution is considered as a cause of a high residual value of TBA. A graph in Non Patent Literature 8 also shows that the higher the substrate concentration is, the more likely the layer separation is caused. Furthermore, Non Patent Literature 8 discloses that, when tobramycin is freeze-dried using 6 to 9% TBA, the higher the TBA concentration is, the higher the residual TBA concentration tends to be, and the cause of the high residual TBA concentration is due to the layer separation of the solution. Further, in Non Patent Literature 9, PC-1, which is a peptide synthesized by Bristol-Myers Squibb (BMS) Company, is dissolved in a mixed solvent of 20-70% tBuOH/$H_2O$ and the mixture is freeze-dried at a substrate concentration of 5 to 10 mg/mL, but freeze-drying is not carried out at a high substrate concentration. Non Patent Literature 10 describes preparing a monophase solution, using a higher concentration than the concentration at which crystals are formed, as the TBA concentration, and selecting a suitable type of cyclodextrin and a suitable freeze-drying process to reduce the residual TBA concentration, but Non Patent Literature 10 neither discloses nor suggests the substrate concentration.

### Citation List

#### Non Patent Literature

Non Patent Literature 1: Biopolymers. 2000; 55(3):227-250.
Non Patent Literature 2: Journal of Pharmaceutical Machinery and Engineering 2015 Vo1.24, No.2, pages 15, 39.
Non Patent Literature 3: European Journal of Pharmaceutical Sciences 15 (2002) 115-133.
Non Patent Literature 4: Journal of Pharmaceutical Sciences 107 (2018) 2005-2012.
Non Patent Literature 5: Journal of Pharmaceutical Sciences 107 (2018) 887-896.
Non Patent Literature 6: Journal of Pharmaceutical Sciences 108 (2019) 399-415.
Non Patent Literature 7: ICHQ3C guideline; Q3C(R8) impurities: Guideline for Residual Solvents, Endorsed on 25 March 2020.

Non Patent Literature 8: Journal of Pharmaceutical Sciences, Vol.91, (2002) 1147-1155.
Non Patent Literature 9: Pharmaceutical Research, Vol.25, No.12, December 2008, 2799-2806.
Non Patent Literature 10: Acta Pharmaceutical Sinica 2007, 42(3) 314-317.

Summary of Invention

Technical Problem

As described above, it is known that, in conventional freeze-drying, the higher the substrate concentration is, the more difficult the control of the residual solvent concentration is (Non Patent Literature 4). Actually, the inventors have applied a lipophilic peptide to the existing TBA-water system freeze-drying method, and as a result, found that the residual solvent concentration should be improved. However, in the case of freeze-drying of the drug substance which is considered to be produced in a large scale, a low substrate concentration causes a reduction in efficiency of freeze-drying. Therefore, in particular, in an industrially large scale, it is desired that freeze-drying can be carried out under further high substrate concentration conditions. In the present invention, an object is to provide a medicine production method including a freeze-drying method that can reduce the residual solvent concentration in a wide substrate concentration range from low concentration to high concentration.

Solution to Problem

The present inventors have extensively conducted studies to reduce the residual solvent concentration, and surprisingly found that the residual solvent concentration can be significantly reduced by performing freeze-drying using a mixed solvent in which an organic solvent and water are mixed in a specific ratio, even when the concentration of the substance to be freeze-dried falls within a wide concentration range from low concentration to high concentration.
That is, the present invention relates to the followings.

[1] A medicine production method including the following steps:

(1) providing a mixed solution containing water, an organic solvent, and a substance to be freeze-dried; and
(2) subjecting the mixed solution to freeze-drying,

wherein a weight percentage of the organic solvent based on a total weight of water and the organic solvent in the step (1) is 68% by weight or more and 97% by weight or less.
[2] A method for freeze-drying a substance to be freeze-dried including the following steps:

(1) providing a mixed solution containing water, an organic solvent, and a substance to be freeze-dried; and
(2) subjecting the mixed solution to freeze-drying,

wherein a weight percentage of the organic solvent based on a total weight of water and the organic solvent in the step (1) is 68% by weight or more and 99% by weight or less.
[3] The method according to [1] or [2], wherein the step (1) includes providing a mixed solvent containing water and the organic solvent, and mixing the mixed solvent and the substance to be freeze-dried.
[4] The method according to any one of [1] to [3], wherein a weight percentage of the substance to be freeze-dried in a volume of the mixed solution containing water, the organic solvent, and the substance to be freeze-dried is 2 w/v% or more and 20 w/v% or less.
[5] The method according to any one of [1] to [4], wherein the step (2) includes a pre-freezing step.
[6] The method according to any one of [1] to [5], wherein the step (2) includes a drying step having a plurality of stages.
[7] The method according to any one of [1] to [6], wherein a freezing point of the mixed solvent containing water and the organic solvent is -60°C or more.
[8] The method according to any one of [1] to [7], wherein the organic solvent is t-butanol.
[9] The method according to any one of [1] to [8], wherein the substance to be freeze-dried is a lipophilic peptide.
[10] The method according to [9], wherein the lipophilic peptide has a cyclic structure.
[11] The method according to [9] or [10], wherein a CLogP of the lipophilic peptide is 25 or less.
[12] The method according to any one of [9] to [11], wherein a molecular weight of the lipophilic peptide is 5,000 or less.
[13] The method according to any one of [9] to [12], wherein a number of amino acid residues of the lipophilic peptide is 5 or more and 30 or less.
[14] The method according to any one of [9] to [13], wherein the lipophilic peptide contains a non-natural amino acid residue.

[15] The method according to [14], wherein the non-natural amino acid residue is a non-natural N-substituted amino acid residue.

[16] The method according to any one of [1] to [15], wherein a residual solvent concentration in a freeze-dried powder obtained by freeze-drying is 1.2% or less.

Advantageous Effects of Invention

According to the present invention, the residual solvent concentration can be significantly reduced in a wide substrate concentration range from low concentration to high concentration by performing freeze-drying using a mixed solvent in which an organic solvent and water are mixed in a specific ratio.

Description of Embodiments

Unless otherwise indicated herein, the abbreviations used herein are listed below.

2-MeTHF: 2-methyltetrahydrofuran
BEP: 2-bromo-1-ethylpyridinium tetrafluoroborate
CPME: cyclopentyl methyl ether
DIPEA: N,N-diisopropylethylamine
DMAP: 4-dimethylaminopyridine
DMC: dimethyl carbonate
HATU: O-(7-azabenzotriazol-1-yl)-N,N,N',N'-tetramethyluronium hexafluorophosphate
HMDS: 1,1,1,3,3,3-hexamethyldisilazane
$LiBH_4$: lithium borohydride
MeCN: acetonitrile
MeOH: methanol
MTBE: methyl tert-butyl ether
NMI: N-methylimidazole
Pd/C: palladium on carbon
PyBOP: 1H-benzotriazole-1-yloxytripyrrolidinophosphonium hexafluorophosphate
T3P: propylphosphonic anhydride
TBAF: tetrabutylammonium fluoride
TFA: trifluoroacetic acid
THF: tetrahydrofuran
TMSOTf: trimethylsilyl trifluoromethanesulfonate

Terms as used herein

As used herein, the "substance to be freeze-dried" refers to a compound to be freeze-dried. In the present invention, the compound to be freeze-dried is preferably an active ingredient of the medicine in the present specification, and is more preferably a low molecular compound or a peptide compound. As used herein, the term "substrate" is used in the same meaning as the "substance to be freeze-dried". As used herein, the composition to be frozen and/or dried may be referred to as the "sample".

As used herein, the term "low molecular compound" refers to a compound preferably having a molecular weight of 2,000 or less other than the peptide compound described below, and examples thereof include natural compounds and compounds that include chemical synthesis in the production process. The molecular weight of the low molecular compound is more preferably 1,000 or less, further preferably 800 or less, and particularly preferably 500 or less. The CLogP of the low molecular compound is preferably 16 or less, more preferably 10 or less, further preferably 7 or less, still further preferably 6 or less, particularly preferably 5 or less, and most preferably 4 or less. The ClogP is a partition coefficient calculated with a computer by dividing a compound into partial structures (the calculation software thereof is known, and for example, the calculation can be performed using a software of Daylight Chemical Information Systems, Inc. or the like).

As used herein, the term "peptide compound" is not particularly limited, as long as it is a peptide compound in which natural amino acids and/or non-natural amino acids are linked by an amide bond or an ester bond. It is desirably a peptide compound preferably having 5 residues or more, more preferably 7 residues or more, further preferably 8 residues or more, and particularly preferably 9 residues or more, and preferably having 30 residues or less, more preferably 25 residues or less, further preferably 15 residues or less, and particularly preferably 13 residues or less. Further, the peptide compound may be a peptide compound preferably having 5 residues or more and 30 residues or

less, more preferably 7 residues or more and 25 residues or less, further preferably 8 residues or more and 15 residues or less, and particularly preferably 9 residues or more and 13 residues or less.

The peptide compound that can be used in the present invention preferably includes at least 3 N-substituted amino acids, and more preferably at least 5 or more N-substituted amino acids in one peptide. These N-substituted amino acids may be present continuously or discontinuously in the peptide compound. The peptide compound in the present invention may be linear or cyclic, and a cyclic peptide compound is preferable. The peptide compound may have a branched structure. Among peptide compounds having a non-natural type amino acid residue, the non-natural type amino acid residue is further preferably a non-natural type N-substituted amino acid residue (also referred to as the "non-natural N-substituted amino acid residue").

As used herein, the "cyclic peptide compound" is not particularly limited, as long as it is a peptide compound having a cyclic portion composed of 5 or more amino acid residues. The cyclization method thereof is also not limited, but it is a cyclic peptide compound obtained by cyclizing a group on the N-terminal side and a group on the C terminal side of a linear peptide compound. The cyclization may take any form, such as cyclization with a carbon-nitrogen bond such as an amide bond, cyclization with a carbon-oxygen bond such as an ester bond or ether bond, cyclization with a carbon-sulfur bond such as a thioether bond, cyclization with a carbon-carbon bond, or cyclization by heterocyclic construction. Among these, cyclization through covalent bonds such as amide bonds or carbon-carbon bonds is preferred, and cyclization through an amide bond by a carboxy group of the side chain and an amino group of the main chain of the N terminal is more preferred. The positions of the carboxy group, the amino group, and the like used for cyclization may be on the main chain or the side chain, and are not particularly limited as long as the positions allow the groups to be cyclized.

As used herein, the term "amino acid" includes a natural amino acid and a non-natural amino acid (sometimes also referred to as an amino acid derivative). As used herein, the term "natural amino acid" refers to Gly, Ala, Ser, Thr, Val, Leu, Ile, Phe, Tyr, Trp, His, Glu, Asp, Gln, Asn, Cys, Met, Lys, Arg, or Pro. The non-natural amino acid (amino acid derivative) is not particularly limited, and examples thereof include a $\beta$-amino acid, a D-type amino acid, an N-substituted amino acid (excluding Pro), an $\alpha,\alpha$-disubstituted amino acid, an amino acid having a side chain different from that of natural amino acids, and a hydroxycarboxylic acid. As the amino acid in the present specification, amino acids having arbitrary conformation are acceptable. The selection of a side chain of an amino acid is not particularly limited, and the side chain is freely selected from, in addition to a hydrogen atom, an alkyl group, an alkenyl group, an alkynyl group, an aryl group, a heteroaryl group, an aralkyl group, a heteroaralkyl group, a cycloalkyl group, a spiro-bonded cycloalkyl group, and the like. Each of the side chains may have a substituent. The substituent is also not limited, and one or two or more substituents may be freely selected independently from arbitrary substituents including, for example, a halogen atom, an O atom, a S atom, a N atom, a B atom, a Si atom, or a P atom. That is, examples of the side chain include an alkyl group, an alkoxy group, an alkenyl group, an alkynyl group, an aryl group, a heteroaryl group, an aralkyl group, or a cycloalkyl group which may be substituted, and oxo, aminocarbonyl, and a halogen atom. In a non-limiting aspect, the amino acid as used herein may be a compound having a carboxy group and an amino group in the same molecule (even in this case, the amino acid also includes imino acids such as proline and hydroxyproline).

Examples of halogen-derived substituents include fluoro (-F), chloro (-C1), bromo (-Br), and iodo (-I).

Examples of O atom-derived substituents include hydroxy (-OH), oxy (-OR), carbonyl (-C=O-R), carboxy ($-CO_2H$), oxycarbonyl (-C=O-OR), carbonyloxy (-O-C=O-R), thiocarbonyl (-C=O-SR), a carbonylthio group (-S-C=O-R), aminocarbonyl (-C=O-NHR), carbonylamino (-NH-C=O-R), oxycarbonylamino (-NH-C=O-OR), sulfonylamino ($-NH-SO_2-R$), aminosulfonyl ($-SO_2-NHR$), sulfamoylamino ($-NH-SO_2-NHR$), thiocarboxy (-C(=O)-SH), and carboxylcarbonyl ($-C(=O)-CO_2H$).

Examples of the oxy (-OR) include alkoxy, cycloalkoxy, alkenyloxy, alkynyloxy, aryloxy, heteroaryloxy, and aralkyloxy.

Examples of the carbonyl (-C=O-R) include formyl (-C=O-H), alkylcarbonyl, cycloalkylcarbonyl, alkenylcarbonyl, alkynylcarbonyl, arylcarbonyl, heteroarylcarbonyl, and aralkylcarbonyl.

Examples of the oxycarbonyl (-C=O-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.

Examples of the carbonyloxy (-O-C=O-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of the thiocarbonyl (-C=O-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of the carbonylthio (-S-C=O-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of the aminocarbonyl (-C=O-NHR) include alkylaminocarbonyl, cycloalkylaminocarbonyl, alkenylaminocarbonyl, alkynylaminocarbonyl, arylaminocarbonyl, heteroarylaminocarbonyl, and aralkylaminocarbonyl. Examples thereof additionally include compounds in which the H atom bonded to the N atom in -C=O-NHR is further replaced with alkyl, cycloalkyl, alkenyl, alkynyl, aryl, heteroaryl, or aralkyl.

Examples of the carbonylamino (-NH-C=O-R) include alkylcarbonylamino, cycloalkylcarbonylamino, alkenylcarbonylamino, alkynylcarbonylamino, arylcarbonylamino, heteroarylcarbonylamino, and aralkylcarbonylamino. Examples thereof additionally include compounds in which the H atom bonded to the N atom in -NH-C=O-R is further replaced with alkyl, cycloalkyl, alkenyl, alkynyl, aryl, heteroaryl, or aralkyl.

Examples of the oxycarbonylamino (-NH-C=O-OR) include alkoxycarbonylamino, cycloalkoxycarbonylamino, alkenyloxycarbonylamino, alkynyloxycarbonylamino, aryloxycarbonylamino, heteroaryloxycarbonylamino, and aralkyloxycarbonylamino. Examples thereof additionally include compounds in which the H atom bonded to the N atom in -NH-C=O-OR is further replaced with alkyl, cycloalkyl, alkenyl, alkynyl, aryl, heteroaryl, or aralkyl.

Examples of the sulfonylamino (-NH-$SO_2$-R) include alkylsulfonylamino, cycloalkylsulfonylamino, alkenylsulfonylamino, alkynylsulfonylamino, arylsulfonylamino, heteroarylsulfonylamino, and aralkylsulfonylamino. Examples thereof additionally include compounds in which the H atom bonded to the N atom in -NH-$SO_2$-R is further replaced with alkyl, cycloalkyl, alkenyl, alkynyl, aryl, heteroaryl, or aralkyl.

Examples of the aminosulfonyl (-$SO_2$-NHR) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. Examples thereof additionally include compounds in which the H atom bonded to the N atom in -$SO_2$-NHR is further replaced with alkyl, cycloalkyl, alkenyl, alkynyl, aryl, heteroaryl, or aralkyl.

Examples of the sulfamoylamino (-NH-$SO_2$-NHR) include alkylsulfamoylamino, cycloalkylsulfamoylamino, alkenylsulfamoylamino, alkynylsulfamoylamino, arylsulfamoylamino, heteroarylsulfamoylamino, and aralkylsulfamoylamino. The two H atoms bonded to the N atoms in -NH-$SO_2$-NHR may be substituted by substituents each independently selected from the group consisting of alkyl, cycloalkyl, alkenyl, alkynyl, aryl, heteroaryl, and aralkyl, and these two substituents may form a ring.

Examples of S atom-derived substituents include thiol (-SH), thio (-S-R), sulfinyl (-S=O-R), sulfonyl (-S(O)$_2$-R), sulfo (-$SO_3H$), and pentafluorosulfanyl (-$SF_5$).

Examples of the thio (-S-R) that can be selected include alkylthio, cycloalkylthio, alkenylthio, alkynylthio, arylthio, heteroarylthio, and aralkylthio.

Examples of the sulfinyl (-S=O-R) include alkylsulfinyl, cycloalkylsulfinyl, alkenylsulfinyl, alkynylsulfinyl, arylsulfinyl, heteroarylsulfinyl, and aralkylsulfinyl.

Examples of the sulfonyl (-S(O)$_2$-R) include alkylsulfonyl, cycloalkylsulfonyl, alkenylsulfonyl, alkynylsulfonyl, arylsulfonyl, heteroarylsulfonyl, and aralkylsulfonyl.

Examples of N atom-derived substituents include azido (-$N_3$, also referred to as "azide group"), cyano (-CN), primary amino (-$NH_2$), secondary amino (-NH-R), tertiary amino (-NR(R')), amidino (-C(=NH)-$NH_2$), substituted amidino (-C(=NR)-NR'R"), guanidino (-NH-C(=NH)-$NH_2$), substituted guanidino (-NR-C(=NR")-NR'R"), and aminocarbonylamino (-NR-CO-NR'R").

Examples of the secondary amino (-NH-R) include alkylamino, cycloalkylamino, alkenylamino, alkynylamino, arylamino, heteroarylamino, and aralkylamino.

Examples of the tertiary amino (-NR(R')) include an amino group having arbitrary two substituents each independently selected from alkyl, cycloalkyl, alkenyl, alkynyl, aryl, heteroaryl, aralkyl, and the like, such as alkyl(aralkyl)amino, and arbitrary two substituents of these may form a ring.

Examples of the substituted amidino (-C(=NR)-NR'R") include groups in which three substituents R, R', and R" on the N atoms are each independently selected from alkyl, cycloalkyl, alkenyl, alkynyl, aryl, heteroaryl, and aralkyl, for example, alkyl(aralkyl)(aryl)amidino.

Examples of the substituted guanidino (-NR-C(=NR‴)-NR'R") include groups in which R,R', R", and R‴ are each independently selected from alkyl, cycloalkyl, alkenyl, alkynyl, aryl, heteroaryl, and aralkyl, and groups in which these substituents form a ring.

Examples of the aminocarbonylamino (-NR-CO-NR'R") include groups in which R, R', and R" are each independently selected from a hydrogen atom, alkyl, cycloalkyl, alkenyl, alkynyl, aryl, heteroaryl, and aralkyl, and groups in which these substituents form a ring.

Examples of B atom-derived substituents include boryl (-BR(R')) and dioxyboryl (-B(OR)(OR')). The two substituents R and R' may be each independently selected from alkyl, cycloalkyl, alkenyl, alkynyl, aryl, heteroaryl, aralkyl, or the like, or may form a ring. Specific examples include a cyclic boryl group, and more specifically include a pinacolate boryl group, a neopentanediolate boryl group, and a catecholate boryl group.

Specific examples of the substituent on the nitrogen atom of the N-substituted amino acid, as used herein include alkyl, $C_1$-$C_6$ alkyl, $C_1$-$C_4$ alkyl, methyl, $C_7$-$C_{14}$ aralkyl, benzyl, and phenethyl.

The backbone amino group of the amino acid may be unsubstituted (-$NH_2$) or may be substituted (i.e., -NHR wherein R represents alkyl, alkenyl, alkynyl, aryl, heteroaryl, aralkyl, or cycloalkyl optionally having a substituent, and a carbon chain bonded to a N atom and a carbon atom at position $\alpha$ may form a ring, as in proline). As used herein, such an amino acid having the substituted backbone amino group may be referred to as the "N-substituted amino acid". Examples of the "N-substituted amino acid" as used herein include preferably, but are not limited to, N-alkylamino acid,

N-$C_1$-$C_6$ alkylamino acid, N-$C_1$-$C_4$ alkylamino acid, N-methylamino acid, N-$C_7$-$C_{14}$ aralkylamino acid, N-benzylamino acid, and N-phenethylamino acid.

As used herein, the "amino acid" includes all isotopes corresponding to each amino acid. The isotope of the "amino acid" is one in which at least one atom is substituted with an atom having the same atomic number (the same number of protons) and a different mass number (different sum of numbers of protons and neutrons). Examples of the isotope included in the "amino acid" as used herein include a hydrogen atom, a carbon atom, a nitrogen atom, an oxygen atom, a phosphorus atom, a sulfur atom, a fluorine atom, and a chlorine atom which respectively include $^{2}H$, $^{3}H$, $^{13}C$, $^{14}C$, $^{15}N$, $^{17}O$, $^{18}O$, $^{32}P$, $^{35}S$, $^{18}F$, and $^{36}Cl$.

As used herein, the "room temperature" means an environment in which the set temperature of an air conditioner is set to 22°C, and is used in the usual meaning in the present technical field without particular limitation. Unless otherwise indicated, the room temperature is, for example, preferably 1 to 30°C, and more preferably about 15 to 28°C.

Medicine production method and method for freeze-drying substance to be freeze-dried

The medicine production method of the present invention includes the following steps:

(1) providing a mixed solution containing water, an organic solvent, and a substance to be freeze-dried; and
(2) subjecting the mixed solution to freeze-drying,

wherein a weight percentage of the organic solvent based on a total weight of water and the organic solvent in step (1) is 68% by weight or more and 99% by weight or less.

A method for freeze-drying a substance to be freeze-dried of the present invention includes the following steps:

(1) providing a mixed solution containing water, an organic solvent, and a substance to be freeze-dried; and
(2) subjecting the mixed solution to freeze-drying,

wherein a weight percentage of the organic solvent based on a total weight of water and the organic solvent in step (1) is 68% by weight or more and 99% by weight or less.

In the method of the present invention, a low molecular compound or a peptide compound can be used as the substance to be freeze-dried. Among these, a lipophilic peptide compound can be preferably used. Further, among lipophilic peptides, a lipophilic peptide having a cyclic structure can be more preferably used.

The CLogP of the lipophilic peptide is preferably 25 or less, more preferably 22 or less, further preferably 20 or less, further preferably 18 or less, further preferably 16 or less, and further preferably 15 or less, and preferably 5 or more, more preferably 10 or more, and further preferably 12 or more. The CLogP of the lipophilic peptide compound is preferably 5 or more and 25 or less, more preferably 10 or more and 20 or less, and further preferably 12 or more and 18 or less.

The molecular weight of the lipophilic peptide is preferably 5,000 or less, more preferably 3,000 or less, and further preferably 2,000 or less, and preferably 500 or more, more preferably 800 or more, and further preferably 1,000 or more. The molecular weight of the lipophilic peptide compound is preferably 500 or more and 5,000 or less, more preferably 800 or more and 3,000 or less, and further preferably 1,000 or more and 2,000 or less.

The "organic solvent" used in the present invention is desired to have a higher vapor pressure as compared with the vapor pressure of water and have a lower latent heat of sublimation as compared with the latent heat of sublimation of water. As the organic solvent, at least one solvent selected from the group consisting of t-butanol, dimethyl sulfoxide, 1,4-dioxane, acetonitrile, acetic acid, cyclohexane, and dimethyl carbonate can be preferably used, at least one solvent selected from the group consisting of t-butanol, dimethyl sulfoxide, and acetonitrile can be more preferably used, and further preferably, it is desired to use t-butanol.

In the organic solvent, the freezing point under atmospheric pressure is preferably -50°C or more, more preferably 0°C or more, further preferably 10°C or more, and particularly preferably 20°C or more, and preferably 50°C or less, more preferably 40°C or less, and further preferably 30°C or less.

The freezing point under atmospheric pressure of the mixed solvent containing the organic solvent and water is preferably -60°C or more, more preferably -40°C or more, further preferably -20°C or more, and particularly preferably -10°C or more, and preferably 10°C or less, more preferably 0°C or less, and further preferably -5°C or less. The freezing point under atmospheric pressure of the mixed solvent containing the organic solvent and water is more preferably -60°C or more and 10°C or less, further preferably -20°C or more and 0°C or less, and particularly preferably -10°C or more and -5°C or less.

When the mixed solvent has a plurality of freezing points, the above range is the preferred range of the lowest freezing point. The freezing point can be determined by subjecting the mixed solvent of the organic solvent and water prepared in advance so as to have a predetermined ratio to measurement by DSC (differential scanning calorimeter).

Specifically, the freezing point can be determined by the method described in Examples.

In the preparation of the mixed solution containing water, an organic solvent, and a substance to be freeze-dried in step (1), the order of mixing of water, the organic solvent, and the substance to be freeze-dried is not particularly limited. Preferably, the mixed solution can be prepared by providing a mixed solvent containing water and an organic solvent, and mixing the mixed solvent and a substance to be freeze-dried. Alternatively, the mixed solution can be prepared by adding a substance to be freeze-dried to an organic solvent to provide a mixed solution of the organic solvent and the substance to be freeze-dried, and adding water to the mixed solution of the organic solvent and the substance to be freeze-dried.

With respect to the weight percentage of the substance to be freeze-dried in the volume of the mixed solution containing water, the organic solvent, and the substance to be freeze-dried, the lower limit value is preferably 2.0 w/v% or more, 5.0 w/v% or more, 7.0 w/v% or more, 9.0 w/v% or more, or 10.0 w/v% or more, and the upper limit value is preferably 20.0 w/v% or less, 15.0 w/v% or less, or 12.0 w/v% or less. The weight percentage of the substance to be freeze-dried may be within a range of an arbitrary combination of these, and for example, is preferably 2.0 w/v% or more and 20.0 w/v% or less, more preferably 5.0 w/v% or more and 15.0 w/v% or less, and further preferably 9.0 w/v% or more and 12.0 w/v% or less.

With respect to the weight percentage of the organic solvent based on the total weight of water and the organic solvent in step (1), the lower limit value is preferably 68% by weight or more, 70% by weight or more, 75% by weight or more, 80% by weight or more, 85% by weight or more, 88% by weight or more, or 90% by weight or more, and the upper limit value is preferably 99% by weight or less, 97% by weight or less, 95% by weight or less, 92% by weight or less, or 90% by weight or less. The weight percentage of the organic solvent based on the total weight of water and the organic solvent in step (1) may be within a range of an arbitrary combination of these, and for example, the weight percentage is preferably 68% by weight or more and 99% by weight or less, more preferably 68% by weight or more and 97% by weight or less, further preferably 80% by weight or more and 95% by weight or less, and still further preferably 88% by weight or more and 92% by weight or less. A freeze-dried product having a significantly reduced residual solvent concentration can be obtained by carrying out freeze-drying using water and the organic solvent in such a weight percentage, not only in the case where the substance to be freeze-dried is present at a low concentration, but also in the case where the substance to be freeze-dried is present at a high concentration.

"Freeze-drying" used in step (2) is a process of sublimating the solvent in a sample in a frozen state to remove the solvent. More specifically, freeze-drying in the present invention is carried out by freezing a mixed solution, and then reducing the ambient pressure such that the frozen water and the frozen organic solvent in the sample are allowed to directly sublimate from the solid phase to the gas phase. The method for carrying out freeze-drying is known to those skilled in the art, and a known freeze-drying apparatus can be used. For example, a shelf freeze dryer, a spray freeze dryer, and a multi-manifold freeze dryer can be used, and preferably, a shelf freeze dryer can be used.

In the present invention, freeze-drying may include a pre-freezing step. Further, freeze-drying may include a drying step having a plurality of stages such as primary drying and secondary drying.

In the pre-freezing step, the mixed solution is cooled to be frozen. The pre-freezing step can be preferably carried out under atmospheric pressure.

The cooling temperature in the pre-freezing step is not particularly limited by the type and proportion of the organic solvent to be used, but is preferably -80°C or more, and more preferably -60°C or more, and preferably -10°C or less, and more preferably -30°C or less. The cooling temperature is preferably -80°C or more and -10°C or less, and more preferably -60°C or more and -30°C or less.

The cooling rate upon cooling is not particularly limited, and the sample can be cooled to a predetermined cooling temperature at a cooling rate of preferably 0.1°C/minute or more and 10°C/minute or less, and more preferably 1°C/minute or more and 5°C/minute or less.

After reaching the predetermined cooling temperature, the sample can be frozen by keeping the predetermined temperature for preferably 30 minutes or more and 10 hours or less, and more preferably 1 hour or more and 5 hours or less.

In the drying step, the frozen sample is dried under reduced pressure. The boiling point of the solvent is lowered by reducing the pressure, and the solvent in the sample is sublimated to be removed from the sample, thereby drying the sample. To supply the energy required upon sublimation, the sample may be heated after reducing the pressure.

The pressure applied to the sample in the freeze-drying apparatus in the drying step is preferably 1 Pa or more and 100 Pa or less, and more preferably 10 Pa or more and 50 Pa or less.

The drying temperature in drying of the sample that may be carried out after reducing the pressure is not particularly limited by the type and proportion of the organic solvent to be used, but the temperature is preferably -60°C or more, and more preferably -50°C or more, and preferably 60°C or less, and more preferably 50°C or less. For example, the temperature upon drying that may be carried out after reducing the pressure is preferably within a temperature range of -60°C or more and 60°C or less, and more preferably -50°C or more and 50°C or less.

The heating rate (temperature rising rate) upon heating to allow the sample to reach the above predetermined drying temperature is not particularly limited by the type and proportion of the organic solvent to be used, but the heating

rate is preferably 0.1°C/minutes or more, and more preferably 1°C/minutes or more, and preferably 10°C/minutes or less, and more preferably 5°C/minutes or less. For example, the sample can be heated to the predetermined drying temperature at a heating rate of preferably 0.1 °C/minutes or more and 10°C/minutes or less, and more preferably about 1°C/minutes or more and 5°C/minutes or less.

The drying step may be carried out in a plurality of stages. When the drying step is divided into two stages, drying can be performed in the first stage at a temperature of preferably -60°C or more, and more preferably -30°C or more, and at a temperature of preferably 0°C or less. For example, the drying temperature in the first stage is preferably within a temperature range of -60°C or more and 0°C or less, and more preferably -30°C or more and 0°C or less.

Further, drying can be performed in the second stage at a temperature of preferably 0°C or more, and more preferably 20°C or more, and at a temperature of preferably 60°C or less, and more preferably 50°C or less. For example, the drying temperature in the second stage is preferably within a range of 0°C or more and 60°C or less, and more preferably 20°C or more and 50°C or less.

In each stage of the first stage and the second stage, the heating rate in the heating for drying of the sample is not particularly limited, but the sample can be heated to the predetermined drying temperature preferably at a heating rate of 0.1°C/minute or more, and more preferably 1°C/minute or more, and preferably 10°C/minute or less, and more preferably 5°C/minute or less. The sample can be heated to the predetermined drying temperature at a heating rate of, for example, preferably 0.1°C/minutes or more and 10°C/minutes or less, and more preferably about 1°C/minutes or more and 5°C/minutes or less.

After reaching the predetermined temperature, in each of the first stage and the second stage, the sample can be dried by keeping the predetermined drying temperature for preferably 12 hours or more and 72 hours or less, and more preferably 24 hours or more and 50 hours or less.

The residual solvent concentration in the freeze-dried powder obtained by freeze-drying is preferably 1.2% or less, more preferably 1% or less, further preferably 0.8% or less, and particularly preferably 0.6% (w/w) or less. The residual solvent concentration in the freeze-dried powder can be measured by dissolving the freeze-dried powder which is the sample in a suitable solvent to obtain a sample solution, and subjecting the sample solution to gas chromatography. Specifically, the residual solvent concentration can be measured by the method described in Examples.

All references cited herein are incorporated herein by reference.

## Examples

Hereinafter, preferred specific aspects of the present invention are described by way of Examples, but the present invention is not limited thereto.

### [Example 1]

[Preparation of substrate solution]

After 1.0 g of ciclosporin A ("ciclosporin A", manufactured by ZHEJIANG RUIBANG LABORATORIES; molecular weight: 1202.61, CLogP: 14.36, number of amino acid residues: 11), and 8 mL of a mixed solvent of tertiary butyl alcohol (TBA, "t-butanol", manufactured by FUJIFILM Wako Pure Chemical Corporation, freezing point: about 25°C) and water (weight ratio of TBA:water = 68:32) were mixed at a room temperature, the mixed solution was shaken at room temperature at 100 rpm for 30 minutes using a shaking apparatus ("small rotary shaker", manufactured by Nisshin Rika Co., Ltd.) to dissolve the ciclosporin A. The obtained mixed solution was diluted with the TBA-water mixed solvent to 10 mL, thereby preparing a 10% (w/v) mixed solution.

[Preliminary freezing]

After 6.5 mL of the prepared mixed solution was injected into a stainless tray (W: 23 mm, D: 32 mm, H: 38 mm), the stainless tray was installed on the shelf in the dryer in a freeze drier ("VirTis AdVantage Pro ADP-B2EL-EOG-X", manufactured by SP SCIENTIFIC) under atmospheric pressure. Subsequently, the temperature in the freeze drier was cooled to -45°C at a cooling rate of 1°C/min and the temperature was kept for 2 hours.

[Primary drying]

After preliminary freezing, the pressure in the freeze drier was reduced to 150 mTorr (about 20 Pa), and the temperature was raised from -45°C to -25°C at a temperature rising rate of 1°C/min and kept. The time from the start of the temperature rise including the time kept at - 25°C was 48 hours.

[Secondary drying]

After primary drying, while maintaining the pressure in the primary drying, the temperature in the freeze drier was raised from -25°C to 40°C at a temperature rising rate of 1 °C/min and kept. The time from the start of the temperature rise including the time kept at 40°C was 48 hours.

[Pretreatment for measurement of residual solvent concentration]

Nitrogen gas was introduced into the freeze drier to bring the pressure back to atmospheric pressure, thereby obtaining a freeze-dried powder which is a sample for measuring the residual solvent concentration. 20.5 mg was weighed from the obtained freeze-dried powder and prepared into a 2 mL solution with 1,3-dimethyl-2-imidazolidinone (DMI, "1,3-dimethyl-2-imidazolidinone", manufactured by FUJIFILM Wako Pure Chemical Corporation), which was used as the sample solution for measuring the residual solvent concentration.

[Measurement of residual solvent concentration]

The residual solvent concentration in the sample solution was measured by gas chromatography under the following conditions.
As the residual solvent standard solution, a solution obtained by diluting 26.7 mg of TBA with 2 mL of DMI was used.
- Apparatus: GC-2010 Plus (SHIMADZU)
- Column: DB-624 (Agilent Technologies)
  0.53 mm ID × 30 m, 3 μm film thickness
- Injection volume: 2 μL
- Column temperature: The temperature was maintained at 40°C for 5 minutes, and then raised to 220°C at a temperature rising rate of 10°C/min. Further, the temperature was maintained at the same temperature for 5 minutes.
- Inlet temperature: 200°C
- Detector: FID
- Detector temperature: 230°C
- Carrier gas: helium (linear velocity: 35 cm/s)
- Split ratio: 1:10
- Retention time of TBA: 1.30 min

The residual solvent concentration was calculated in accordance with the following expression 1 and expression 2 using the peak area of the chromatogram obtained by gas chromatography.

(Expression 1)

$$\text{TBA concentration in sample solution (mg/mL)} = \frac{\text{TBA concentration in residual solvent standard solution} \times \text{residual solvent area value of sample solution}}{\text{Residual solvent area value of residual solvent standard solution}}$$

(Expression 2)

$$\text{Residual solvent concentration in sample (\%)} = \frac{\text{TBA concentration in sample solution}}{\text{Sample concentration in sample solution}} \times 100$$

The residual solvent concentrations obtained by the expression 2 are shown in Table 1.

[Examples 2 to 11 and Comparative Examples 1 to 4]

The residual solvent concentrations were measured in the same manner as in Example 1, except that the

amount of ciclosporin A used in the preparation of the substrate solution, the weight percentage of TBA based on the total weight of TBA and water, the freezing point of the mixed solvent of TBA and water in each TBA weight percentage (the lowest freezing point when the mixed solvent had a plurality of freezing points), the substrate concentration when the mixed solution was diluted with the TBA-water mixed solvent to 10 mL, and the amount of the freeze-dried powder weighed in the pretreatment for measurement of the residual solvent concentration were changed as described in Table 1. The results are shown in Table 1.

The freezing point of the mixed solvent of TBA and water in each TBA weight percentage was measured by the following method (provided that, the freezing point when the TBA weight percentage is 100% is the catalog value of the manufacturer).

[Measurement of freezing point]

About 3 μL of the mixed solvent of TBA and water which was prepared in advance with each TBA weight percentage was dispensed into a SUS sealed container (SUS sealed sample container 15μL, manufactured by Hitachi High-Tech Science Corporation) using a micropipette (Rainin Pos-D positive displacement pipette, manufactured by METTLER TOLEDO), the SUS container was sealed with a sample sealer (electric sample sealer, manufactured by Hitachi High-Tech Science Corporation), and the freezing point was measured using a thermal analysis apparatus (DSC 3+, manufactured by METTLER TOLEDO).

Equipment used: thermal analysis apparatus DSC 3+, manufactured by METTLER TOLEDO

Measurement conditions: The temperature was cooled from 30°C to -60°C at a cooling rate of -5°C/min and maintained at the same temperature for 20 minutes. Subsequently, the temperature was raised from -60°C to 30°C at a temperature rising rate of 1°C/min.

[Table 1]

| | Ciclosporin A (g) | TBA ratio (mass ratio) | Freezing point (°C) | Substrate concentration (w/v%) | Freeze-dried powder (mg) | Residual solvent concentration (%) |
|---|---|---|---|---|---|---|
| Example 1 | 1.0 | 68 | about -10 | 10 | 20.5 | 1.00 |
| Example 2 | 1.0 | 70 | about -10 | 10 | 21.0 | 0.64 |
| Example 3 | 1.0 | 75 | about -7 | 10 | 20.6 | 0.80 |
| Example 4 | 1.0 | 90 | about -7 | 10 | 20.3 | 0.52 |
| Example 5 | 1.0 | 95 | about -7 | 10 | 20.1 | 0.54 |
| Example 6 | 1.0 | 97 | about -7 | 10 | 20.3 | 0.61 |
| Example 7 | 0.2 | 90 | about -7 | 2 | 27.2 | 0.12 |
| Example 8 | 2.0 | 90 | about -7 | 20 | 23.8 | 0.94 |
| Example 9 | 0.2 | 68 | about -10 | 2 | 24.1 | 0.30 |
| Example 10 | 2.0 | 97 | about -7 | 20 | 21.4 | 1.08 |
| Example 11 | 0.2 | 97 | about -7 | 2 | 18.5 | 0.28 |
| Comparative Example 1 | 1.0 | 40 | about -10 | 10 | 20.2 | 1.95 |
| Comparative Example 2 | 1.0 | 60 | about -10 | 10 | 21.0 | 1.43 |
| Comparative Example 3 | 1.0 | 65 | about -10 | 10 | 19.9 | 1.81 |
| Comparative Example 4 | 1.0 | 100 | about 25 | 10 | 19.7 | 2.00 |

[Synthetic Examples] Synthesis of cyclic peptide compound 1

The analysis conditions by HPLC were shown below.

HPLC analysis conditions, method 1
Apparatus: Waters ACQUITY UPLCH-Class
Column: Ascentis Express 90A C18 (Sigma-Aldrich), 2.1 mm ID × 50 mm, 2.7 μm
Mobile phase: 0.05% TFA/water (A), 0.05% TFA/MeCN (B)
Elution method: B) 5% (0 min) → 100% (5 min) → 5% (5.1 min) → 5% (7 min)
Flow rate: 0.5 mL/min
Column temperature: 35°C
Detection wavelength: 210 nm (PDA (photodiode array))
HPLC analysis conditions, method 3
Apparatus: Waters ACQUITY UPLCH-Class
Column: CAPCELL CORE ADME (OSAKA SODA), 2.1 mm ID × 50 mm, 2.7 μm
Mobile phase: 0.05% TFA/water (A), 0.05% TFA/MeCN (B)
Elution method: B): 5%(0 min) → 100% (5 min) → 5% (5.1 min) → 5% (7 min)
Flow rate: 0.5 mL/min
Column temperature: 35°C
Detection wavelength: 210 nm (PDA)
HPLC analysis conditions, method 4
Apparatus: Waters ACQUITY UPLCH-Class
Column: ACQUITY UPLC CSH C18 (Waters), 2.1 mm ID × 100 mm, 1.7 μm
Mobile phase: 0.05% TFA/water (A), 0.05% TFA/MeCN (B)
Elution method: B) 20% (0 min) → 100% (10 min) → 100% (13.5 min) → 20% (13.6 min) → 20% (18.0 min)
Flow rate: 0.3 mL/min
Column temperature: 50°C
Detection wavelength: 210 nm (PDA)
HPLC analysis conditions, method 5
Apparatus: Waters ACQUITY UPLCH-Class
Column: ACQUITY UPLC CSH C18 (Waters), 2.1 mm ID × 150 mm, 1.7 μm
Mobile phase: 0.05% TFA/water (A), 0.05% TFA/MeCN (B)
Elution method: B) 20% (0 min) → 100% (24 min) → 100% (29 min) → 20% (29.1 min) → 20% (34 min)
Flow rate: 0.3 mL/min
Column temperature: 50°C
Detection wavelength: 220 nm (PDA)

Analysis conditions of HPLC used for each compound are shown in Table 2.

[Table 2]

| Step (Compound number) | HPLC analysis conditions | Retention time (min) | MS Found(m/z) | MS polarity |
|---|---|---|---|---|
| Step H' 1 (Compound a03) | method 3 | 4.499 | 447 | $[M+H]^+$ |
| Step H' 2-1 (Compound a04) | method 3 | 2.419 | 313 | $[M+H]^+$ |
| Step H' 2-2 (Compound a06) | method 3 | 4.458 | 540 | $[M+Na]^+$ |
| Step H' 3-1 (Compound a07) | method 3 | 2.848 | 384 | $[M+H]^+$ |
| Step H' 3-2 (Compound a09) | method 3 | 4.055 | 611 | $[M+Na]^+$ |
| Step H' 4-1 (Compound a10) | method 3 | 2.521 | 455 | $[M+H]^+$ |
| Step H' 4-2 (Compound a12) | method 1 | 4.006 | 703 | $[M+H]^+$ |
| Step H' 5-1 (Compound a13) | method 1 | 2.776 | 569 | $[M+H]^+$ |
| Step H' 5-2 (Compound a15) | method 1 | 4.919 | 840 | $[M+H]^+$ |
| Step H' 6 (Compound a16) | method 1 | 2.909 | 696 | $[M+H]^+$ |
| Step S' 0 (Compound a19) | method 3 | 3.934 | 405 | $[M+H]^+$ |
| Step S' 1-1 (Compound a20) | method 3 | 2.058 | 271 | $[M+H]^+$ |
| Step S' 1-2 (Compound a22) | method 3 | 4.428 | 518 | $[M+H]^+$ |
| Step S' 2-1 (Compound a23) | method 3 | 2.393 | 384 | $[M+H]^+$ |

(continued)

| Step (Compound number) | HPLC analysis conditions | Retention time (min) | MS Found(m/z) | MS polarity |
|---|---|---|---|---|
| Step S' 2-2 (Compound a25) | method 3 | 4.002 | 613 | $[M+Na]^+$ |
| Step S' 3-1 (Compound a26) | method 3 | 2.868 | 517 | $[M+Na]^+$ |
| Step S' 3-2 (Compound a28) | method 3 | 4.323 | 748 | $[M+Na]^+$ |
| Step S' 4-1 (Compound a29) | method 3 | 2.970 | 592 | $[M+H]^+$ |
| Step S' 4-2 (Compound a31) | method 3 | 4.794 | 910 | $[M+Na]^+$ |
| Step 1' (Compound a32) | method 3 | 4.001 | 831 | $[M+H]^+$ |
| Step 2' (Compound a33) | method 4 | 10.65 | 1531 | $[M+Na]^+$ |
| Step 3' (Compound a34) | method 4 | 9.26 | 1474 | $[M+Na]^+$ |
| Step 4' (Compound a35) | method 4 | 12.39 | 1319 | $[M+H]^+$ |
| Step 5' (Compound a36) | method 5 | 18.69 | 1300 | $[M+H]^+$ |

Synthetic Example 1: Step H'1

Compound a03: synthesis of tert-butyl 2-[[(2S)-2-[benzyloxycarbonyl(methyl)aminol-3-cyclohexyl-propanoyl]-methyl-amino]acetate

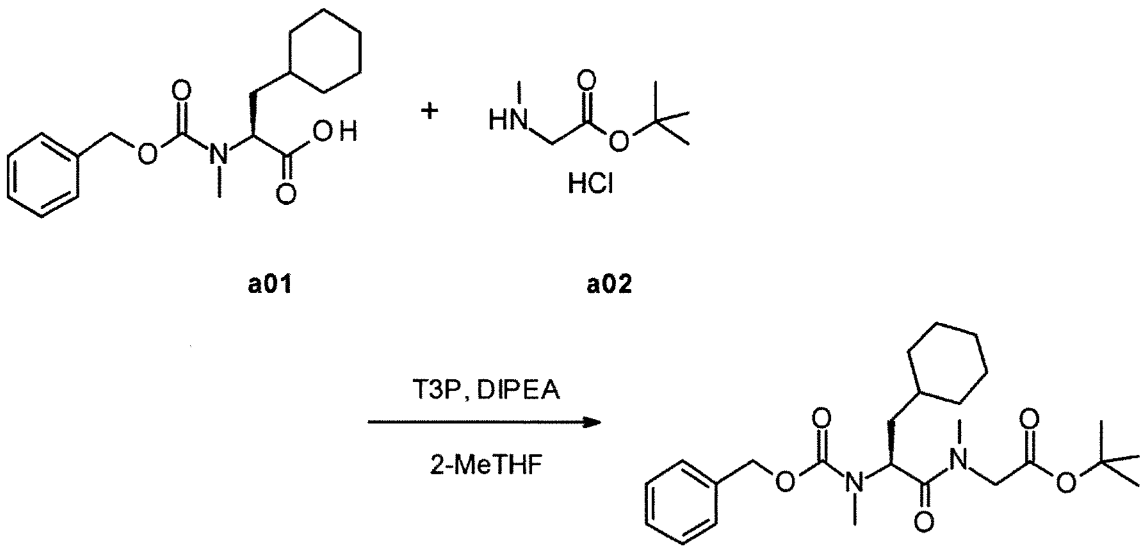


a01 a02

a03

A compound a01 (2.00 g) and a compound a02 (1.37 g) were added to a reaction vessel, 2-MeTHF (19.0 mL) was then added, and the mixture was stirred. Further, after DIPEA (5.5 mL) was added, T3P (50 w/w% 2-MeTHF solution, 11.7 mL) was added dropwise while keeping the internal temperature of the reaction mixture at 32°C or less, and then the mixture was stirred at room temperature for 1 hour. An 5% aqueous sodium carbonate solution (12 mL) was added dropwise such that the internal temperature of the reaction mixture was kept at 36°C or less, followed by stirring. Then, the aqueous layer was discharged. The obtained organic layer was washed with a 5% aqueous sodium carbonate solution (12 mL × 1), a 5% aqueous sodium hydrogen sulfate monohydrate solution (12 mL × 1), and a 10% aqueous sodium chloride solution (50 mL × 2). The obtained organic layer was concentrated under reduced pressure to obtain a residue (2.72 g) containing the compound a03.
The retention time by HPLC analysis: 4.499 minutes (HPLC analysis conditions: method 3)

Synthetic Example 2: Step H'2-1

Compound a04: synthesis of tert-butyl 2-[[(2S)-3-cyclohexyl-2-(methylamino)propanoyl]-methyl-amino]acetate

a03

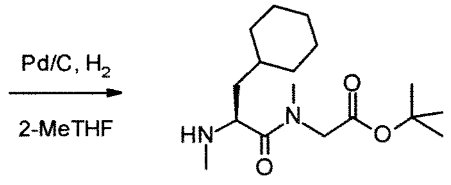


a04

After 2-MeTHF (18 mL) was added to the residue (2.70 g) containing the compound a03 obtained in Step H'1, 5% Pd/C (1.27 g, STD type 50% hydrous product, manufactured by N.E. CHEMCAT CORPORATION) was added. Degassing and replacement with hydrogen gas were carried out for three times, and then the mixture was stirred for 2 hours. The reaction mixture was suction filtered using a filter paper (40 mm, No. 5C, manufactured by Kiriyama glass. CO.), and the residue was washed with a 2-MeTHF solution (18 mL × 3). The obtained filtrate and washing solution were concentrated together under reduced pressure to obtain a residue (1.77 g) containing the compound a04.

The retention time by HPLC analysis: 2.419 minutes (HPLC analysis conditions: method 3)

Synthetic Example 3: Step H'2-2

Compound a06: synthesis of tert-butyl 2-[[(2S)-2-[[2-[benzyloxycarbonyl(methyl)amino]acetyl]-methyl-amino]-3-cyclohexyl-propanoyl]-methyl-amino]acetate

a04 +

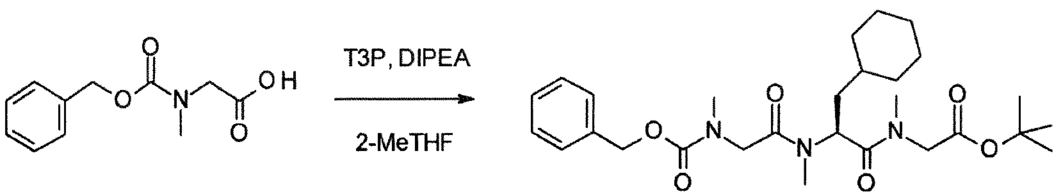


a05

a06

The residue (1.71 g) containing the compound a04 obtained in Step H'2-1 and a compound a05 (1.29 g) were dissolved in 2-MeTHF (11.6 mL) and stirred. Further, after DIPEA (3.4 mL) was added, T3P (50 w/w% 2-MeTHF solution, 7.2 mL) was added dropwise while keeping the internal temperature of the reaction mixture at 27°C or less, and then the mixture was stirred at room temperature for 2 hours. An 5% aqueous sodium carbonate solution (7.2 mL) was added dropwise such that the internal temperature of the reaction mixture was kept at 29°C or less, followed by stirring. Then, the aqueous layer was discharged. The obtained organic layer was washed with a 5% aqueous sodium carbonate solution (7.2 mL × 1), a 5% aqueous sodium hydrogen sulfate monohydrate solution (7.2 mL × 1), and a 10% aqueous sodium chloride solution (7.2 mL × 2). The obtained organic layer was concentrated under reduced pressure to obtain a residue (2.70 g) containing the compound a06.

The retention time by HPLC analysis: 4.458 minutes (HPLC analysis conditions: method 3)

Synthetic Example 4: Step H'3-1

Compound a07: synthesis of tert-butyl 2-[[(2S)-3-cyclohexyl-2-[methyl-[2-(methylamino)acetyl]amino]propanoyl]-methyl-amino]acetate

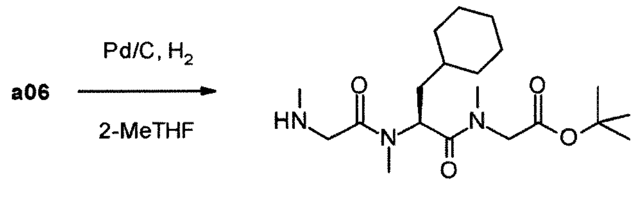


**a07**

After 2-MeTHF (10 mL) was added to the residue (2.70 g) containing the compound a06 obtained in Step H'2-2, 5% Pd/C (0.71 g, STD type 50% hydrous product, manufactured by N.E. CHEMCAT CORPORATION) was added. Degassing and replacement with hydrogen gas were carried out for three times, and then the mixture was stirred for 2 hours. The reaction mixture was suction filtered using a filter paper (40 mm, No. 5C, manufactured by Kiriyama glass. CO.), and the residue was washed with a 2-MeTHF solution (10 mL × 3). The obtained filtrate and washing solution were concentrated together under reduced pressure to obtain a residue (1.82 g) containing the compound a07.

The retention time by HPLC analysis: 2.848 minutes (HPLC analysis conditions: method 3)

Synthetic Example 5: Step H'3-2

Compound a09: synthesis of tert-butyl 2-[[(2S)-2-[[2-[[2-[benzyloxycarbonyl(methyl)amino]acetyl]-methyl-amino]acetyl]-methyl-amino]-3-cyclohexyl-propanoyl]-methyl-amino]acetate

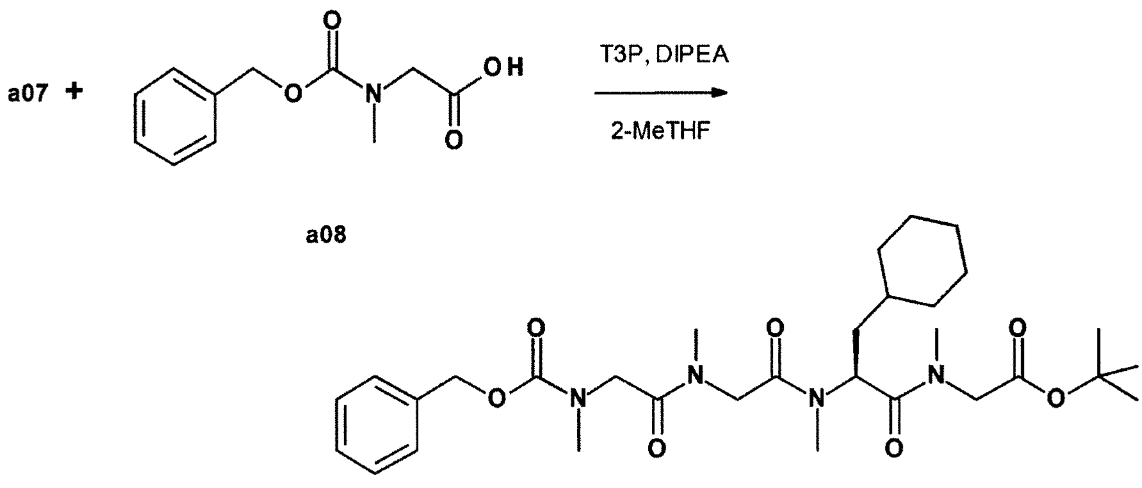


**a09**

The residue (1.80 g) containing the compound a07 obtained in Step H'3-1 and a compound a08 (1.57 g) were dissolved in 2-MeTHF (14.2 mL) and stirred. Further, after DIPEA (4.1 mL) was added, T3P (50 w/w% 2-MeTHF solution, 8.8 mL) was added dropwise while keeping the internal temperature of the reaction mixture at 30°C or less, and then the mixture was stirred at room temperature for 2 hours. An 5% aqueous sodium carbonate solution (10.8 mL) was added dropwise such that the internal temperature of the reaction mixture was kept at 33°C or less, followed by stirring.

Then, the aqueous layer was discharged. The obtained organic layer was washed with a 5% aqueous sodium carbonate solution (10.8 mL × 1), a 5% aqueous sodium hydrogen sulfate monohydrate solution (10.8 mL × 1), and a 5% aqueous sodium carbonate solution (10.8 mL × 1). The obtained organic layer was concentrated under reduced pressure to obtain a residue (2.61 g) containing the compound a09.

The retention time by HPLC analysis: 4.055 minutes (HPLC analysis conditions: method 3)

Synthetic Example 6: Step H'4-1

Compound a10: synthesis of tert-butyl 2-[[(2S)-3-cyclohexyl-2-[methyl-[2-[methyl-[2-(methylamino)acetyl]amino]acetyl]amino]propanoyl]-methyl-amino]acetate

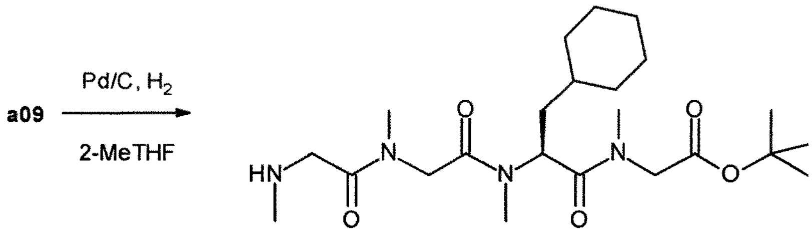

a09

a10

After 2-MeTHF (12.3 mL) was added to the residue (2.40 g) containing the compound a09 obtained in Step H'3-2, 5% Pd/C (0.44 g, STD type 50% hydrous product, manufactured by N.E. CHEMCAT CORPORATION) was added. Degassing and replacement with hydrogen gas were carried out for three times, and then the mixture was stirred for 1 hour. The reaction mixture was suction filtered using a filter paper (40 mm, No. 5C, manufactured by Kiriyama glass. CO.), and the residue was washed with a 2-MeTHF solution (12 mL × 3). The obtained filtrate and washing solution were concentrated together under reduced pressure to obtain a residue (1.97 g) containing the compound a10.

The retention time by HPLC analysis: 2.521 minutes (analysis conditions: method 3)

Synthetic Example 7: Step H'4-2

Compound a12: synthesis of tert-butyl 2-[[(2S)-2-[[2-[[2-[[(2S,3S)-2-(benzyloxycarbonylamino)-3-methyl-pentanoyl]-methyl-amino]acetyl]-methyl-amino]acetyl]-methyl-amino]-3-cyclohexyl-propanoyl]-methyl-amino]acetate

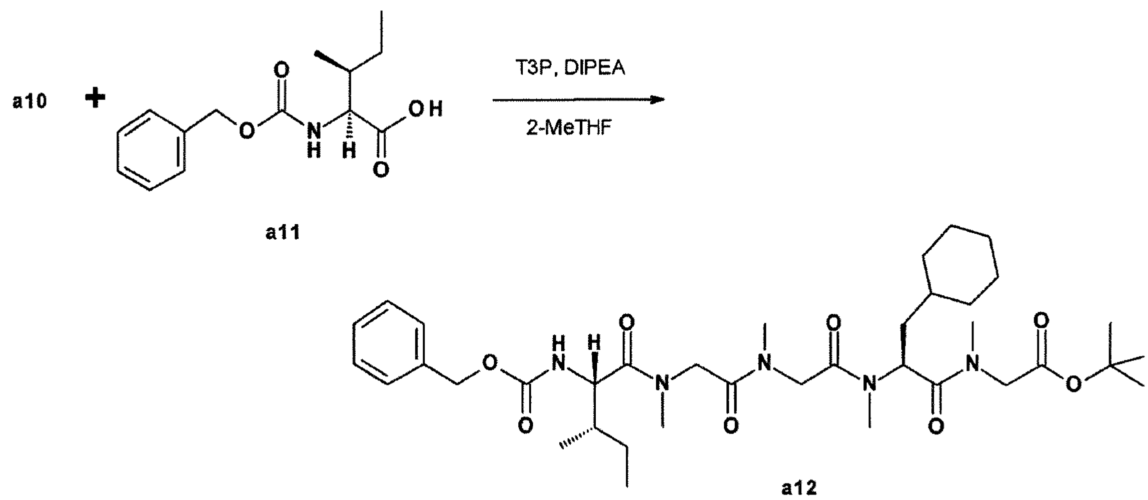

a10 +

a11

a12

The residue (1.92 g) containing the compound a10 obtained in Step H'4-1 and a compound a11 (1.69 g) were dissolved in 2-MeTHF (12.8 mL) and stirred. Further, after DIPEA (3.7 mL) was added, T3P (50 w/w% 2-MeTHF solution, 7.8 mL) was added dropwise while keeping the internal temperature of the reaction mixture at 29°C or less, and then the mixture was stirred at room temperature for 2 hours. An 5% aqueous sodium carbonate solution (14.4 mL) was added dropwise such that the internal temperature of the reaction mixture was kept at 33°C or less, followed by stirring. Then, the aqueous layer was discharged. The obtained organic layer was washed with a 5% aqueous sodium carbonate solution (14.4 mL × 1), a 5% aqueous sodium hydrogen sulfate monohydrate solution (14.4 mL × 1), and a 5% aqueous sodium carbonate solution (14.4 mL × 1). Further, the obtained organic layer was washed with a 5% aqueous sodium hydrogen sulfate monohydrate solution (14.4 mL × 1) and a 5% aqueous sodium carbonate solution (14.4 mL × 1), and this operation was repeated twice. 2-MeTHF (14.4 mL) was added thereto, and this mixture was washed with a 5% aqueous sodium hydrogen sulfate monohydrate solution (14.4 mL × 1) and a 5% aqueous sodium carbonate solution (14.4 mL × 1). Further, this was washed with a 1% aqueous sodium carbonate solution (14.4 mL × 3), a 5% aqueous sodium carbonate solution (14.4 mL × 5), a 5% aqueous sodium hydrogen sulfate monohydrate solution (14.4 mL × 1), a 5% aqueous sodium carbonate solution (14.4 mL × 1), a 5% aqueous sodium hydrogen sulfate monohydrate solution (14.4 mL × 1), and a 5% aqueous sodium carbonate solution (14.4 mL × 1, 7.2 mL × 10). Further, this was washed with 2.5% aqueous ammonia (7.2 mL × 3) and a 10% aqueous sodium chloride solution (1 mL × 1). The obtained organic layer was concentrated under reduced pressure to obtain a residue (2.39 g) containing the compound a12.

The retention time by HPLC analysis: 4.006 minutes (HPLC analysis conditions: method 1)

Synthetic Example 8: Step H'5-1

Compound a13: synthesis of tert-butyl 2-[[(2S)-2-[[2-[[2-[[(2S,3S)-2-amino-3-methyl-pentanoyl]-methyl-amino]acetyl]-methyl-amino]acetyl]-methyl-amino]-3-cyclohexyl-propanoyl]-methyl-amino]acetate

a12

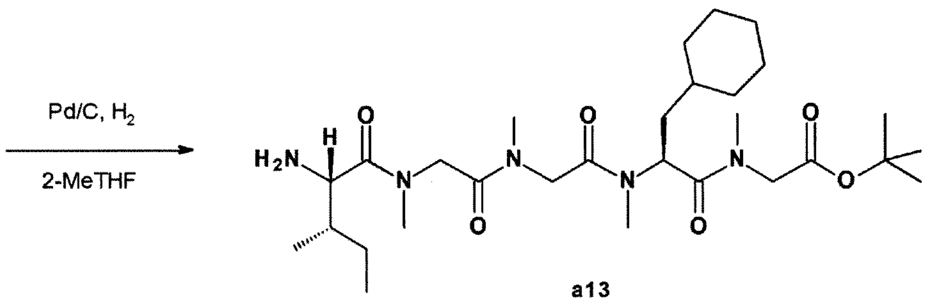


a13

After 2-MeTHF (9.3 mL) was added to the residue (2.15 g) containing the compound a12 obtained in Step H'4-2, 5% Pd/C (0.66 g, STD type 50% hydrous product, manufactured by N.E. CHEMCAT CORPORATION) was added. Degassing and replacement with hydrogen gas were carried out for three times, and then the mixture was stirred for 2 hours. The reaction mixture was suction filtered using a filter paper (40 mm, No. 5C, manufactured by Kiriyama glass. CO.), and the residue was washed with a 2-MeTHF solution (10 mL × 3). The obtained filtrate and washing solution were concentrated together under reduced pressure to obtain a residue (1.95 g) containing the compound a13.

The retention time by HPLC analysis: 2.776 minutes (HPLC analysis conditions: method 1)

Synthetic Example 9: Step H'5-2

Compound a15: synthesis of tert-butyl 2-[[(2S)-3-cyclohexyl-2-[methyl-[2-[methyl-[2-[methyl-[(2S,3S)-3-methyl-2-[[(2S)-4-methyl-2-[methyl(2-trimethylsilylethoxycarbonyl)amino]pentanoyl]amino]pentanoyl]amino]acetyl]amino]acetyl]amino]propanoyl]-methyl-amino]acetate

a13 +

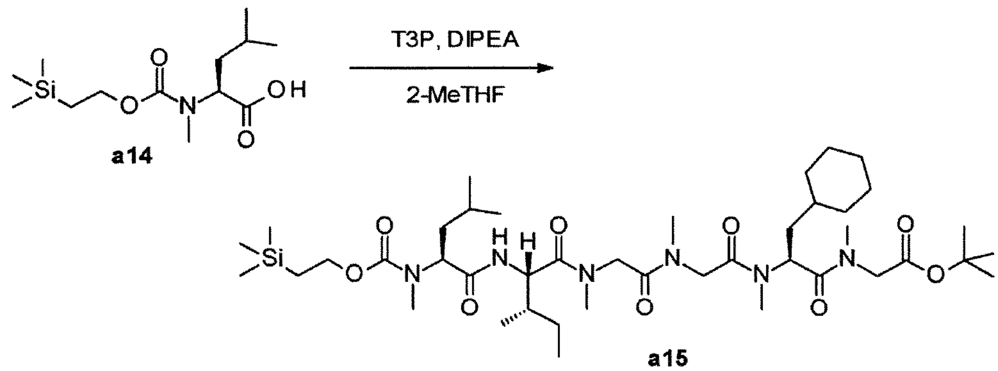


a14

a15

The residue (1.94 g) containing the compound a13 obtained in Step H'5-1 and a compound a14 (1.49 g) were dissolved in 2-MeTHF (10.3 mL) and stirred. The reaction vessel was cooled in an ice bath. After DIPEA (3.0 mL) was added thereto, T3P (50 w/w% 2-MeTHF solution, 6.3 mL) was added dropwise while keeping the internal temperature of the reaction mixture at 9°C or less, and then the mixture was taken out from the ice bath and stirred at room temperature for 1 hour. An 5% aqueous sodium carbonate solution (12 mL) was added dropwise such that the internal temperature of the reaction mixture was kept at 22°C or less, followed by stirring. Then, the aqueous layer was discharged. The obtained organic layer was washed with a 5% aqueous sodium carbonate solution (12 mL × 1), a 5% aqueous sodium hydrogen sulfate monohydrate solution (12 mL × 1), and a 5% aqueous sodium carbonate solution (12 mL × 1), and the obtained organic layer was concentrated under reduced pressure. The organic layer was dissolved again in 2-MeTHF (20 mL) and washed with a 5% aqueous sodium hydrogen sulfate monohydrate solution (12 mL × 2) and a 5% aqueous sodium carbonate solution (12 mL × 2). N-methylimidazole (0.3 mL) and a 5% aqueous sodium carbonate solution (12 mL) were added to the organic layer, the mixture was stirred for 6.5 hours, and then the aqueous layer was discharged. The organic layer was washed with a 5% aqueous sodium carbonate solution (12 mL × 1), a 5% aqueous sodium hydrogen sulfate monohydrate solution (12 mL × 2), and a 5% aqueous sodium carbonate solution (12 mL × 2), and concentrated under reduced pressure. The organic layer was dissolved again in 2-MeTHF (20 mL), a heptane:MTBE mixed solution (1.5:1) (20 mL) was further added thereto, and the mixture was washed with a 5% aqueous sodium carbonate solution (20 mL × 2) and concentrated under reduced pressure to obtain a residue (2.38 g) containing the compound a15.

The retention time by HPLC analysis: 4.919 minutes (HPLC analysis conditions: method 1)

Synthetic Example 10: Step H'6

<u>Compound a16: synthesis of tert-butyl 2-[[(2S)-3-cyclohexyl-2-[methyl-[2-[methyl-[2-[methyl-[(2S,3S)-3-methyl-2-[[(2S)-4-methyl-2-(methylamino)pentanoyl]amino]pentanoyl]amino]acetyl]amino]acetyl]amino]propanoyl]-methyl-amino]acetate</u>

a15

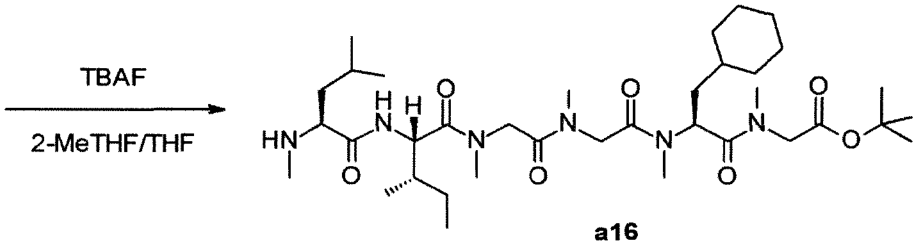


a16

After 2-MeTHF (14 mL) was added to the residue (2.35 g) containing the compound a15 obtained in Step H'5-2, the external temperature of the reaction vessel was set to 50°C, and tetrabutylammonium fluoride (1M THF solution, 7.0 mL) was added. The reaction liquid was stirred as it was for 2 hours. The reaction liquid was cooled to room temperature, isopropyl acetate (7 mL) was then added, and the mixture was washed with a 5% aqueous potassium carbonate solution (7 mL × 6) and then concentrated under reduced pressure to obtain a residue (1.92 g) containing the compound a16.

The retention time by HPLC analysis: 2.909 minutes (HPLC analysis conditions: method 1)

Synthetic Example 11: Step S'0

Compound a19: synthesis of tert-butyl (3S)-3-[benzyloxycarbonyl(methyl)amino]-4-oxo-4-(1-piperidyl)butanoate

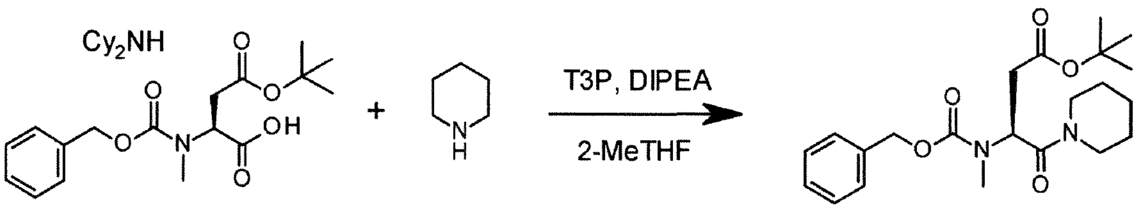


**a17** **a18** **a19**

A compound a17 (2.01 g) and 2-MeTHF (11.7 mL) were added to a reaction vessel and stirred. After DIPEA (1.8 mL) and a compound a18 (0.53 mL) were added, T3P (3.61 mL) was added at room temperature, and then the mixture was stirred for 1 hour. After a 10% aqueous citric acid solution (12 mL) was added while stirring, the aqueous layer was discharged. The obtained organic layer was washed with a 10% aqueous citric acid solution (12 mL × 1) and a 5% aqueous sodium carbonate solution (12 mL × 2). The obtained organic layer was concentrated under reduced pressure to obtain a residue (1.56 g) containing the compound a19. The retention time by HPLC analysis: 3.934 minutes (HPLC analysis conditions: method 3)

Synthetic Example 12: Step S'1-1

Compound a20: synthesis of tert-butyl (3S)-3-(methylamino)-4-oxo-4-(1-piperidyl)butanoate

**a19**

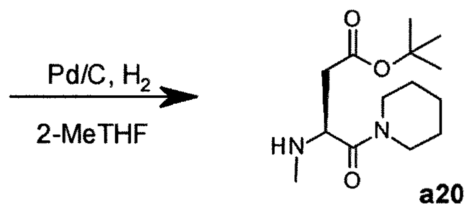


**a20**

After 2-MeTHF (21.3 mL) was added to the residue (3.08 g) containing the compound a19 obtained in Step S'0, 5% Pd/C (4.09 g) was added. Degassing and replacement with hydrogen gas were carried out for three times, and then the mixture was stirred for 2 hours. The reaction mixture was suction filtered using a filter paper (40 mm, No. 5C, manufactured by Kiriyama glass. CO.), and the residue was washed with a 2-MeTHF solution (21.3 mL × 3). The obtained filtrate and washing solution were concentrated together under reduced pressure to obtain a residue (2.09 g) containing the compound a20.

The retention time by HPLC analysis: 2.058 minutes (HPLC analysis conditions: method 3)

Synthetic Example 13: Step S'1-2

Compound a22: synthesis of tert-butyl (3S)-3-[[(2S)-2-[benzyloxycarbonyl(methyl)aminol-3-methyl-butanoyl]-methyl-amino]-4-oxo-4-(1-piperidyl)butanoate

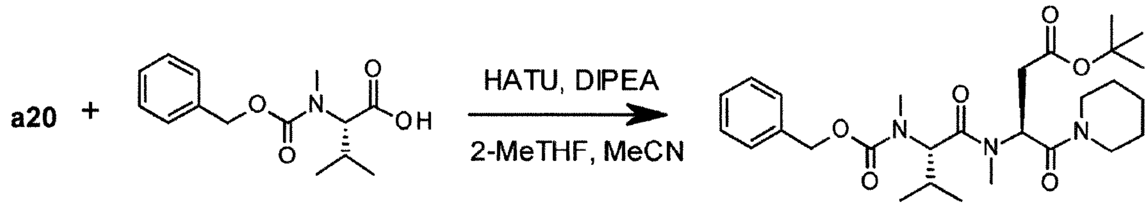


a21

a22

The residue containing the compound a20 (2.04 g) obtained in Step S'1-1 and a compound a21 (2.14 g) were dissolved in 2-MeTHF (6.3 mL) and stirred. Further, after DIPEA (5.3 mL) was added, HATU (3.95 g) dissolved in 2-MeTHF (5.9 mL) and MeCN (4.1 mL) was added at room temperature, and then the mixture was stirred at 50°C for 5 hours. After CPME (5.3 mL) was added at room temperature, a 5% aqueous potassium carbonate solution (4.1 mL) and NMI (0.55 mL) were added, and the mixture was stirred for 1 hour 30 minutes. After a 2.5% aqueous ammonium solution (16.3 mL) was added while stirring, the aqueous layer was discharged. The obtained organic layer was washed with a 2.5% aqueous ammonium solution (16.3 mL × 1), a 10% aqueous sodium hydrogen sulfate monohydrate solution (20.4 mL × 4), a 5% aqueous potassium carbonate solution (20.4 mL × 1), a 10% aqueous sodium hydrogen sulfate monohydrate solution (20.4 mL × 3), and a 5% aqueous potassium carbonate solution (20.4 mL × 1). The obtained organic layer was concentrated under reduced pressure to obtain a residue (3.66 g) containing the compound a22.

The retention time by HPLC analysis: 4.428 minutes (HPLC analysis conditions: method 3)

Synthetic Example 14: Step S'2-1

Compound a23: synthesis of tert-butyl (3S)-3-[methyl-[(2S)-3-methyl-2-(methylamino)butanoyl]amino]-4-oxo-4-(1-piperidyl)butanoate

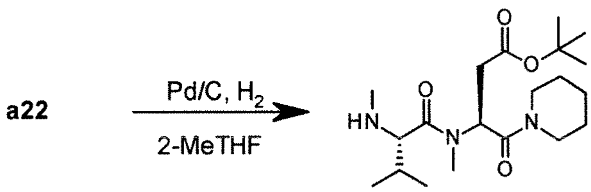


a23

After 2-MeTHF (18.3 mL) was added to the residue (3.56 g) containing the compound a22 obtained in Step S'1-2, 5% Pd/C (2.10 g) was added. Degassing and replacement with hydrogen gas were carried out for three times, and then the mixture was stirred for 2 hours 30 minutes. The reaction mixture was suction filtered using a filter paper (40 mm, No. 5C, manufactured by Kiriyama glass. CO.), and the residue was washed with 2-MeTHF (18.3 mL × 3). The obtained filtrate and washing solution were concentrated together under reduced pressure to obtain a residue (2.58 g) containing the compound a23.

The retention time by HPLC analysis: 2.393 minutes (HPLC analysis conditions: method 3)

Synthetic Example 15: Step S'2-2

Compound a25: synthesis of tert-butyl (3S)-3-[methyl-[(2S)-3-methyl-2-[methyl-[1-[(2,2,2-trifluoroacetyl)amino]cyclopentanecarbonyl]amino]butanoyl]amino]-4-oxo-4-(1-piperidyl)butanoate

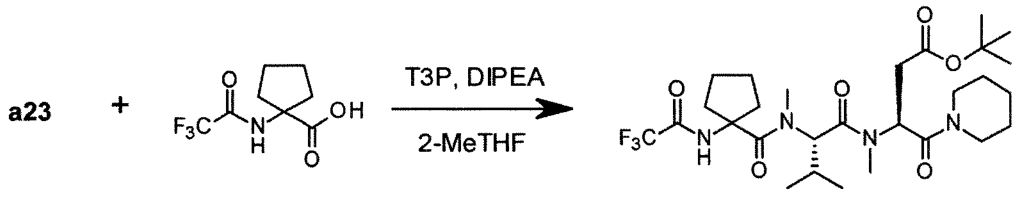


a24

a25

A compound a24 (2.90 g) was dissolved in 2-MeTHF (18.6 mL) and stirred. Further, DIPEA (5.4 mL) and the residue containing the compound a23 (2.50 g) obtained in Step S'2-1 were added, T3P (10.4 mL) and DMAP (1.59 g) were then added at room temperature, and then the mixture was stirred for 8 hours. Each of the compound a24 (1.47 g), DMAP (0.80 g), T3P (5.5 mL), and DIPEA (2.8 mL) was added, and then the mixture was stirred for 2 hours. After a 5% aqueous sodium carbonate solution (20.5 mL) was added while stirring, the aqueous layer was discharged. The obtained organic layer was washed with a 5% aqueous sodium hydrogen sulfate monohydrate solution (20.5 mL × 4), a 5% aqueous sodium carbonate solution (20.5 mL × 2), a 5% aqueous sodium hydrogen sulfate monohydrate solution (20.5 mL × 2), and a 5% aqueous sodium carbonate solution (20.5 mL × 1). The obtained organic layer was concentrated under reduced pressure to obtain a residue (3.45 g) containing the compound a25.

The retention time by HPLC analysis: 4.002 minutes (HPLC analysis conditions: method 3)

Synthetic Example 16: Step S'3-1

Compound a26: synthesis of tert-butyl (3S)-3-[[(2S)-2-[(1-aminocyclopentanecarbonyl)-methyl-amino]-3-methyl-butanoyl]-methyl-amino]-4-oxo-4-(1-piperidyl)butanoate

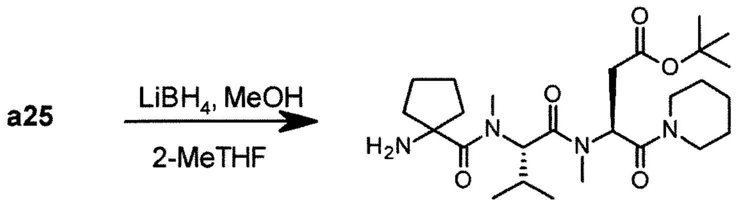


a26

The residue (3.41 g) containing the compound a25 obtained in Step S'2-2 was dissolved in 2-MeTHF (1,362 mL) and MeOH (1.4 mL) and stirred. $LiBH_4$ (ALDRICH, a 2M THF solution, 4.5 mL) was added dropwise at -20°C, and then the mixture was stirred for 2 hours. After 2,2,2-trifluoroethanol (6.4 mL) was added dropwise, the mixture was warmed to 0°C and then stirred for 20 minutes. A 20% aqueous ammonium chloride solution (10.2 mL) was added dropwise, and the aqueous layer was discharged. Trifluoroacetic acid (0.69 mL) was added to the obtained organic layer at room temperature, and then the mixture was stirred for 10 minutes. A reaction solution containing the compound a26 was added dropwise to the reaction vessel containing a 2M aqueous sodium hydroxide solution (44.3 mL). The aqueous layer was discharged, and then the organic layer was washed with a 2M aqueous sodium hydroxide solution (34.1 mL × 2) and a 10% aqueous potassium dihydrogen phosphate solution (17.0 mL × 1). The obtained organic layer was concentrated under reduced pressure to obtain a residue (2.90 g) containing the compound a26.

The retention time by HPLC analysis: 2.868 minutes (HPLC analysis conditions: method 3)

Synthetic Example 17: Step S'3-2

Compound a28: synthesis of benzyl (2S)-2-[[1-[[(1S)-1-[[(1S)-3-tert-butoxy-3-oxo-1-(piperidine-1-carbonyl)propyl]-methyl-carbamoyl]-2-methyl-propyl]-methylcarbamoyl]cyclopentyl]carbamoyl]pyrrolidine-1-carboxylate

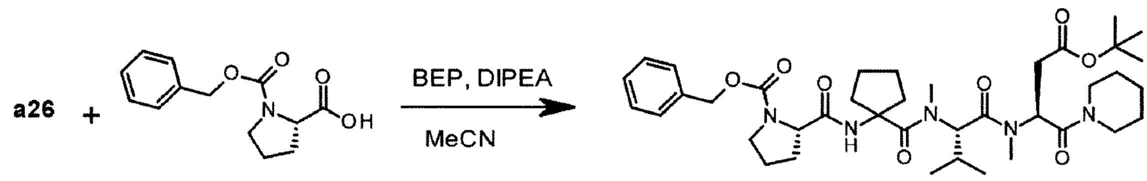


a27

a28

The residue (2.90 g) containing the compound a26 obtained in Step S'3-1 and a compound a27 (1.66 g) were dissolved in MeCN (14.5 mL) and stirred. Further, after DIPEA (2.67 mL) was added, BEP (2.11 g) was added at room temperature, and then the mixture was stirred for 3 hours. After CPME (29.3 mL) was added, a 5% aqueous potassium carbonate solution (17.4 mL) and N-methylimidazole (0.41 mL) were added, and the mixture was stirred for 30 minutes at room temperature. After the aqueous layer was discharged, the obtained organic layer was washed with a 5% aqueous sodium hydrogen sulfate monohydrate solution (17.4 mL × 5), a 5% aqueous sodium carbonate solution (17.4 mL × 2), a 5% aqueous sodium hydrogen sulfate monohydrate solution (17.4 mL × 3), and a 5% aqueous sodium carbonate solution (17.4 mL × 2). The obtained organic layer was concentrated under reduced pressure to obtain a residue (3.86 g) containing the compound a28.

The retention time by HPLC analysis: 4.323 minutes (HPLC analysis conditions: method 3)

Synthetic Example 18: Step S'4-1

Compound a29: synthesis of tert-butyl (3S)-3-[methyl-[(2S)-3-methyl-2-[methyl-[1-[[(2S)-pyrrolidine-2-carbonyl]amino]cyclopentanecarbonyl]amino]butanoyl]amino]-4-oxo-4-(1-piperidyl)butanoate

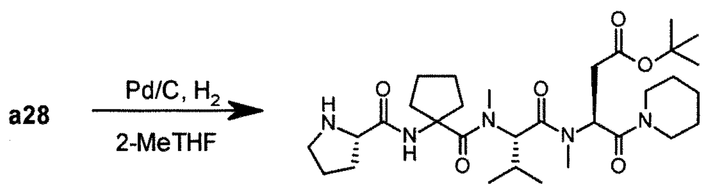


a29

After THF (16.8 mL) was added to the residue (3.81 g) containing the compound a28 obtained in Step S'3-2, 5% Pd/C (0.40 g) was added. Degassing and replacement with hydrogen gas were carried out for three times, and then the mixture was stirred for 4 hours 30 minutes. 5% Pd/C (0.20 g) was added, and then the mixture was stirred for 1 hour 30 minutes. The reaction mixture was suction filtered using a filter paper (40 mm, No. 5C, manufactured by Kiriyama glass. CO.), and the residue was washed with a 2-MeTHF solution (6.6 mL × 3). The obtained filtrate and washing solution were concentrated together under reduced pressure to obtain a residue (3.12 g) containing the compound a29.

The retention time by HPLC analysis: 2.970 minutes (HPLC analysis conditions: method 3)

Synthetic Example 19: Step S'4-2

Compound a31: synthesis of tert-butyl (3S)-3-[[(2S)-2-[[1-[[(2S)-1-[(2S)-2-(benzyloxycarbonylamino)-4-phenyl-butanoyl]pyrrolidine-2-carbonyl]amino]cyclopentanecarbonyl]-methyl-amino]-3-methyl-butanoyl]-methyl-amino]-4-oxo-4-(1-piperidyl)butanoate

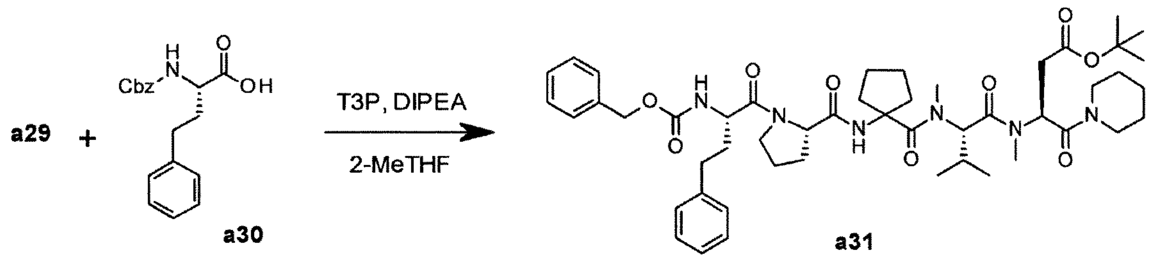


The residue (2.06 g) containing the compound a29 obtained in Step S'4-1 and a compound a30 (1.10 g) were dissolved in 2-MeTHF (10.3 mL) and stirred. Further, after DIPEA (2.8 mL) was added, T3P (5.4 mL) was added at room temperature, and then the mixture was stirred for 2 hours. Each of the compound a30 (0.55 g), DIPEA (1.1 mL), and T3P (2.2 mL) was added, and then the mixture was stirred for 5 hours. Further, the compound a30 (0.57 g), DIPEA (1.1 mL), and T3P (2.2 mL) were added, and the mixture was allowed to stand still overnight and stirred the next day for 2 hours. A 5% aqueous potassium carbonate solution (12.4 mL) and N-methylimidazole (0.29 mL) were added, and the mixture was stirred at room temperature for 3 hours. N-methylimidazole (0.23 mL) was added, the mixture was stirred for 1 hour, and then the aqueous layer was discharged. 2-MeTHF (12.4 mL), N-methylimidazole (0.23 mL), and a 5% aqueous potassium carbonate solution (12.4 mL) were added, the mixture was stirred for 1 hour, and the aqueous layer was discharged. The obtained organic layer was washed with a 10% aqueous sodium hydrogen sulfate monohydrate solution (12.4 mL × 2) and a 5% aqueous potassium carbonate solution (12.4 mL × 1). A mixed solution of heptane and MTBE (heptane/MTBE = 1.5:1, 12.4 mL) and MeCN (4.7 mL) were added to the organic layer, and the aqueous layer was discharged. 2-MeTHF (2.1 mL) was added to the organic layer, which was then washed 7 times with MeCN (7.0 mL) and a 5% aqueous potassium carbonate solution (17.7 mL). Isopropyl acetate (7.6 mL) was added to the organic layer, the mixture was concentrated under reduced pressure, and isopropyl acetate (7.6 mL) was added to the obtained residue to obtain a solution containing the compound a31 (10.31 g).

The retention time by HPLC analysis: 4.794 minutes (HPLC analysis conditions: method 3)

Synthetic Example 20: Step1'

Compound a32: synthesis of (3S)-3-[[(2S)-2-[[1-[[(2S)-1-[(2S)-2-(benzyloxycarbonylamino)-4-phenyl-butanoyl]pyrrolidine-2-carbonyl]amino]cyclopentanecarbonyl]-methyl-amino]-3-methyl-butanoyl]-methyl-amino]-4-oxo-4-(1-piperidyl)butanoic acid

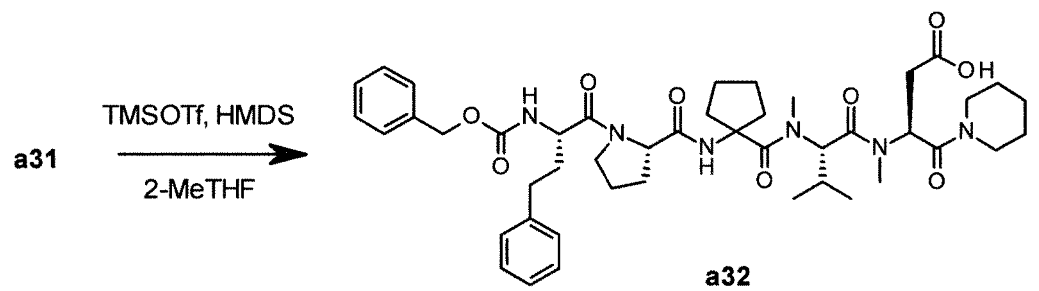


The residue (10.27 g) containing the compound a31 obtained in Step S'4-2 was dissolved in isopropyl acetate (51.4 mL), and the mixture was stirred. After HMDS (2.1 mL) was added, TMSOTf (1.4 mL) was added dropwise at 0°C, and then the mixture was stirred at room temperature for 2 hours 30 minutes. 2-MeTHF (51.4 mL) and a 5% aqueous potassium dihydrogen phosphate solution (102.8 mL) were added at room temperature, and the aqueous layer was discharged. After the organic layer was washed with a 5% aqueous sodium dihydrogen phosphate solution (102.8 mL), DIPEA (3.0 mL) was added to the obtained organic layer, the mixture was concentrated under reduced pressure, and isopropyl acetate (7.6 mL) was added to obtain a solution containing the compound a32 (7.92 g).

The retention time by HPLC analysis: 4.001 minutes (HPLC analysis conditions: method 3)

Synthetic Example 21: Step2'

Compound a33: synthesis of tert-butyl 2-[[(2S)-2-[[2-[[2-[[(2S,3S)-2-[[(2S)-2-[[(3S)-3-[[(2S)-2-[[1-[[(2S)-1-[(2S)-2-(benzyloxycarbonylamino)-4-phenyl-butanoyl]pyrrolidine-2-carbonyl]amino]cyclopentanecarbonyl]-methyl-amino]-3-methyl-butanoyl]-methyl-amino]-4-oxo-4-(1-piperidyl)butanoyl]-methyl-aminol-4-methyl-pentanoyl]amino]-3-methyl-pentanoyl]-methyl-amino]acetyl]-methyl-amino]acetyl]-methyl-amino]-3-cyclohexyl-propanoyl]-methyl-amino]acetate

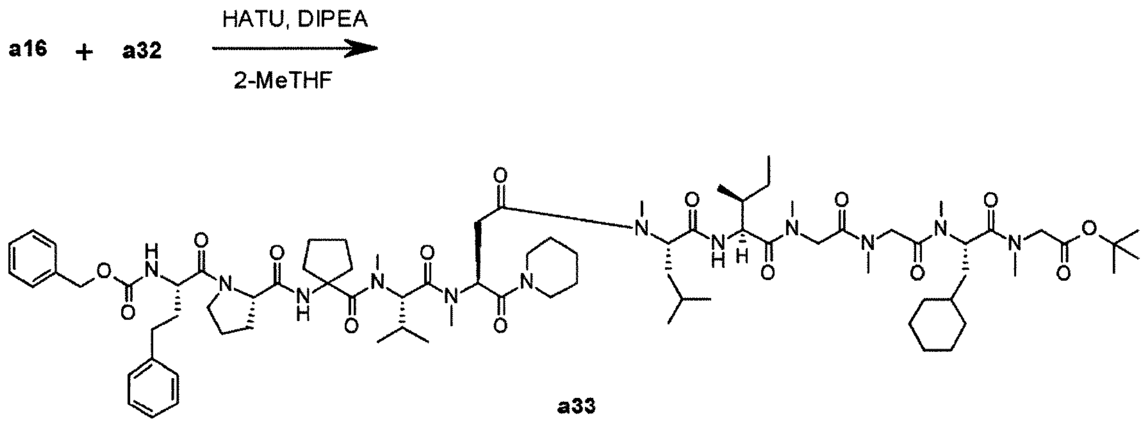


a33

The residue (7.92 g) containing the compound a32 obtained in Step 1' and the residue (1.33 g) containing the compound a16 obtained in Step H'6 were dissolved in 2-MeTHF (4.6 mL), and the mixture was stirred. DIPEA (1.6 mL) and HATU (1.43 g) were added at room temperature, and then the mixture was stirred for 2 hours. The residue (about 300 mg) containing the compound a16 was added, and the mixture was further stirred for 2 hours. Then, the residue (about 300 mg) containing the compound a16 was added, and the mixture was stirred for 1 hour 30 minutes. After HATU (0.79 g) was added, the mixture was stirred for 1 hour. CPME (3.5 mL), N-methylimidazole (0.13 mL), and a 5% aqueous potassium carbonate solution (2.7 mL) were added, and the mixture was stirred at room temperature for 3 hours. After the aqueous layer was discharged, the organic layer was washed with a 2.5% aqueous ammonia solution (9.2 mL × 1), a 10% aqueous sodium hydrogen sulfate monohydrate solution (9.2 mL × 1), a 5% aqueous sodium carbonate solution (9.2 mL × 1), a 10% aqueous sodium hydrogen sulfate monohydrate solution (9.2 mL × 3), and a 5% sodium bicarbonate aqueous solution (9.2 mL × 2). After heptane/MTBE (1.5:1, 9.2 mL) was added to the organic layer, the mixture was washed with a 5% aqueous sodium carbonate solution (9.2 mL × 2). 2-MeTHF (9.2 mL) was added to the obtained organic layer, and the mixture was concentrated under reduced pressure to obtain a residue (4.49 g) containing the compound a33.

The retention time by HPLC analysis: 10.65 minutes (analysis conditions: method 4)

Synthetic Example 22: Step3'

Compound a34: synthesis of 2-[[(2S)-2-[[2-[[2-[[(2S,3S)-2-[[(2S)-2-[[(3S)-3-[[(2S)-2-[[1-[[(2S)-1-[(2S)-2-(benzyloxycarbonylamino)-4-phenyl-butanoyl]pyrrolidine-2-carbonyl]amino]cyclopentanecarbonyl]-methyl-amino]-3-methyl-butanoyl]-methyl-amino]-4-oxo-4-(1-piperidyl)butanoyl]-methyl-amino]-4-methyl-pentanoyl]amino]-3-methyl-pentanoyl]-methyl-amino]acetyl]-methyl-amino]acetyl]-methyl-amino]-3-cyclohexyl-propanoyl]-methyl-amino]acetic acid

a33

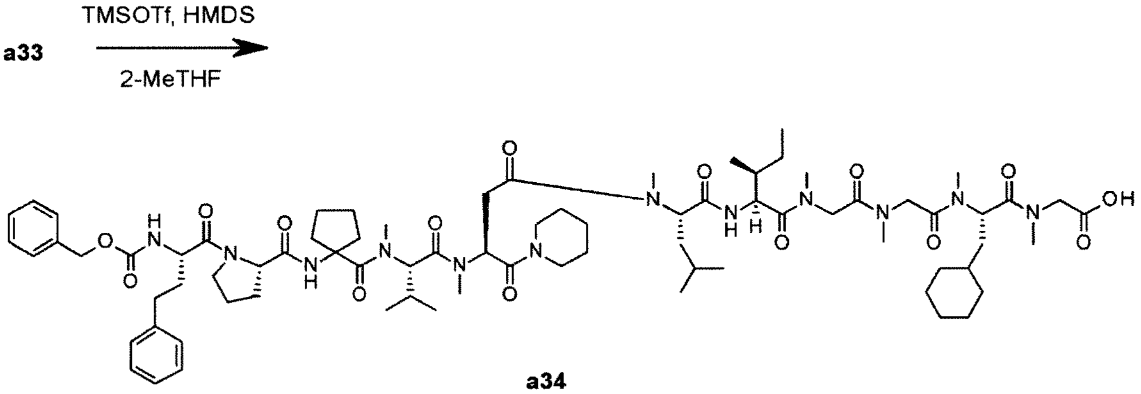


a34

The residue (4.49 g) containing the compound a33 obtained in Step 2' was dissolved in 2-MeTHF (49.5 mL), and the mixture was stirred. HMDS (2.9 mL) and TMSOTf (2.1 mL) were added at room temperature, and then the mixture was stirred for 2 hours. After a 5% aqueous potassium dihydrogen phosphate solution (14.2 mL) was added, the aqueous layer was discharged. The obtained organic layer was washed three times with a mixed aqueous solution of a 10% aqueous citric acid solution (3.4 mL) and a 5% aqueous potassium dihydrogen phosphate solution (10.5 mL), and once with a 5% aqueous sodium carbonate solution. THF (20.9 mL) was added to the organic layer, which was subjected to azeotropic dehydration for three times, and then THF (5.9 mL) was added to the obtained residue to obtain a solution containing the compound a34 (9.59 g).

The retention time by HPLC analysis: 9.26 minutes (analysis conditions: method 4)

Synthetic Example 23: Step 4'

Compound a35: synthesis of 2-[[(2S)-2-[[2-[[2-[[(2S,3S)-2-[[(2S)-2-[[(3S)-3-[[(2S)-2-[[1-[[(2S)-1-[(2S)-2-amino-4-phenylbutanoyl]pyrrolidine-2-carbonyl]amino]cyclopentanecarbonyl]-methyl-amino]-3-methyl-butanoyl]-methyl-amino]-4-oxo-4-(1-piperidyl)butanoyl]-methyl-amino]-4-methyl-pentanoyl]amino]-3-methyl-pentanoyl]-methyl-amino]acetyl]-methyl-amino]acetyl]-methyl-amino]-3-cyclohexyl-propanoyl]-methyl-amino]acetic acid

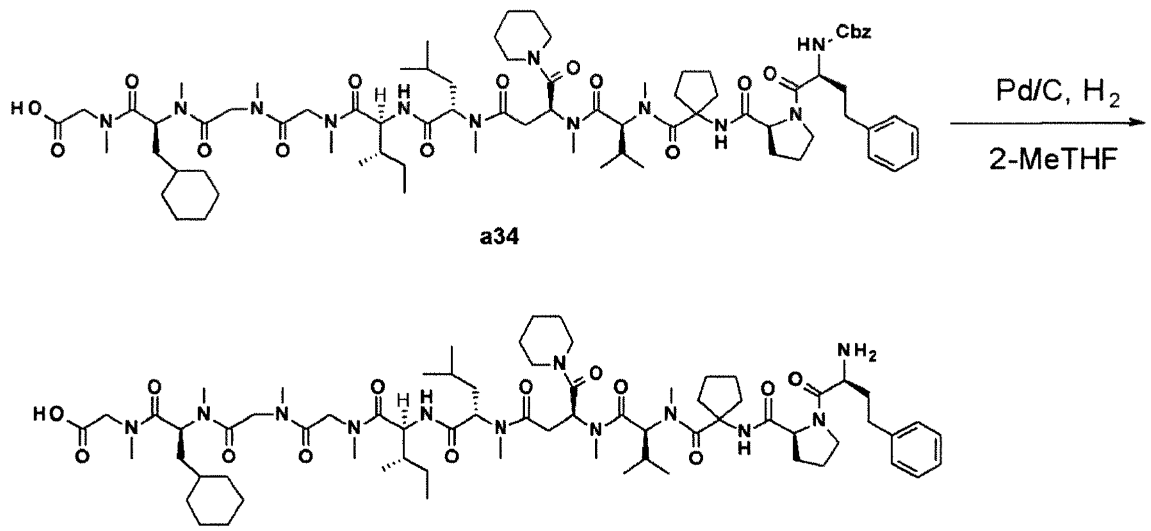


a34

a35

5% Pd/C (0.49 g, STD type 50% hydrous product manufactured by N.E. CHEMCAT CORPORATION) was added to a reaction vessel and suspended in THF (8 mL), and the mixture was stirred under a hydrogen atmosphere for 30 minutes. Then, the mixture was subjected to nitrogen replacement, a solution obtained by dissolving a residue (9.3 g) containing the compound a34 obtained in Step 3' in THF (8 mL) was added, and the mixture was stirred in a hydrogen atmosphere for 6 hours. The reaction conversion rate was 76%. The mixture was subjected to nitrogen replacement, stored in a refrigerator overnight, allowed to return to room temperature the next day, and stirred for 2 hours. The reaction conversion rate was 87%. After the mixture was subjected to nitrogen replacement, a suspension of 5% Pd/C (0.24 g) in THF (4 mL) was added to the reaction liquid, which was subjected to hydrogen replacement and then stirred for 4 hours (the reaction conversion rate was 99.0%). The mixture was subjected to nitrogen replacement, stored in a refrigerator overnight, and allowed to return to room temperature the next day. The reaction conversion rate measured was 99.4%. The reaction mixture was suction filtered using a filter paper (40 mm, No. 5C, manufactured by Kiriyama glass. CO.), and the residue was washed with a 2-MeTHF solution (6.5 mL × 10). The obtained filtrate and washing solution were concentrated together under reduced pressure. The obtained residue was dissolved in acetonitrile (16.3 mL) and 2-MeTHF (6.5 mL), and the mixture was washed with heptane (37.1 mL) and then concentrated. The obtained residue was dissolved again in acetonitrile (16.3 mL) and 2-MeTHF (6.5 mL), and the mixture was washed with heptane (37.1 mL) and then concentrated to obtain a residue (2.96 g) containing the compound a35.

The retention time by HPLC analysis: 12.39 minutes (analysis conditions: method 4)

Synthetic Example 24: Step 5'

Compound a36: synthesis of (3S,9S,18S,21S,25S,28S,34S)-9-(cyclohexylmethyl)-21-isobutyl-28-isopropyl-7,10,13,16,22,26,29-heptamethyl-18-[(1S)-1-methylpropyl]-3-(2-phenylethyl)-25-(piperidine-1-carbonyl)spiro[1,4,7,10,13,16,19,22,26,29,32-undecazabicyclo[32.3.0]heptatriacontane-31,1'-cyclopentane]-2,5,8,11,14,17,20,23,27,30,33-undecone

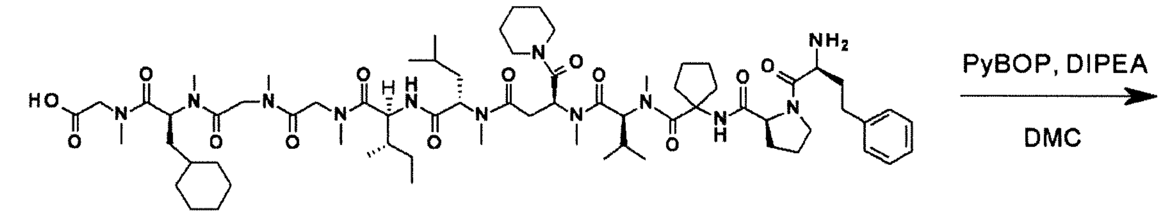


a35

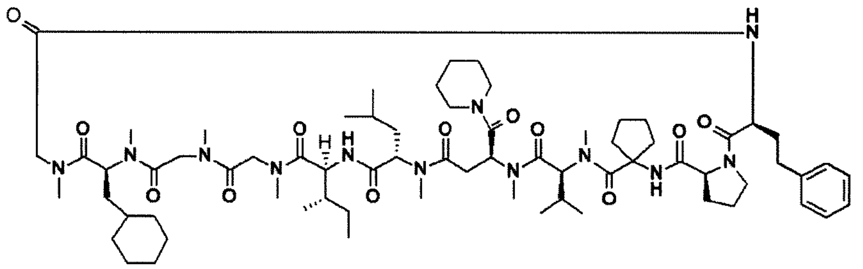

a36

A dimethyl carbonate solution (72.5 mL) containing the residue (2.90 g) containing the compound a35 obtained in the previous step and DIPEA (1.44 mL) was prepared, which was added dropwise to a solution of PyBOP (4.34 g) in dimethyl carbonate (72.5 mL) over 3 hours. Sampling was performed at the time 30 minutes after the completion of the dropwise addition to verify the reaction, insolubles were then filtered by suction filtration using a filter paper (40 mm, No. 5C, manufactured by Kiriyama glass. CO.), and the residue was washed with dimethyl carbonate (15 mL). A solution in which the filtrate and the washing solution were mixed together was washed with a 2.5% aqueous ammonia solution (58 mL), a 5% aqueous potassium hydrogen sulfate solution (58 mL), a 5% aqueous disodium hydrogen phosphate solution (58 mL), a 5% aqueous sodium chloride solution (58 mL), and a 0.5% aqueous sodium chloride solution (58 mL). The obtained organic layer was concentrated under reduced pressure to obtain a residue containing the compound a36 (2.72 g).

The retention time by HPLC analysis: 18.69 minutes (HPLC analysis conditions: method 5)

The obtained residue (2.72 g) was purified by silica gel column chromatography (dichloromethane/methanol) to obtain the compound a36 (1.1 g). The molecular weight of the obtained compound a36 was 1299.71, ClogP was

13.64, and the number of amino acid residues was 11.

[Examples 12 to 16]

The residual solvent concentrations were measured in the same manner as in Example 1, except that the compound a36 was used instead of ciclosporin A, and the amount of the compound a36 used in the preparation of the substrate solution, the weight percentage of TBA based on the total weight of TBA and water, the freezing point of the mixed solvent of TBA and water in each TBA weight percentage (the lowest freezing point when the mixed solvent has a plurality of freezing points), the substrate concentration when the mixed solution was diluted with the TBA-water mixed solvent to 10 mL, and the amount of the freeze-dried powder weighed in the pretreatment for measurement of the residual solvent concentration were changed as described in Table 3. The results are shown in Table 3.

[Table 3]

| | Compound a36 (g) | TBA ratio (mass ratio) | Freezing point (°C) | Substrate concentration (w/v%) | Freeze-dried powder (mg) | Residual solvent concentration (%) |
|---|---|---|---|---|---|---|
| Example 12 | 1 | 90 | about -7 | 10 | 22.4 | 0.14 |
| Example 13 | 0.2 | 97 | about -7 | 2 | 20.0 | 0.17 |
| Example 14 | 0.2 | 68 | about -10 | 2 | 19.5 | 0.25 |
| Example 15 | 0.2 | 90 | about -7 | 2 | 23.5 | 0.14 |
| Example 16 | 0.2 | 99 | about -7 | 2 | 21.4 | 0.29 |

As shown in the above results, a freeze-dried product having a significantly reduced residual solvent concentration can be obtained by carrying out freeze-drying using water and an organic solvent in a specific weight percentage, even in the case where the substance to be freeze-dried is present at a high concentration. For example, when Example 1 and Comparative Example 3, as well as Example 6 and Comparative Example 4 are compared, it is found that the residual solvent concentrations are significantly different from each other, although the TBA/water ratio difference therebetween is slight. Such a result of the residual solvent concentration is conventionally not known, and this is a surprising result that is first found through the study of the present inventors.

Industrial Applicability

In the present invention, the residual solvent concentration can be significantly reduced in a wide substrate concentration range from low concentration to high concentration by performing freeze-drying using a mixed solvent in which an organic solvent and water are mixed in a specific ratio. That is, according to the method of the present invention, the residual solvent concentration can be reduced even when freeze-drying is carried out under high substrate concentration conditions, so that freeze-drying can be efficiency carried out, for example, in a large industrial scale.

## Claims

1. A medicine production method comprising the following steps:

    (1) providing a mixed solution containing water, an organic solvent, and a substance to be freeze-dried; and
    (2) subjecting the mixed solution to freeze-drying,

    wherein a weight percentage of the organic solvent based on a total weight of water and the organic solvent in the step (1) is 68% by weight or more and 99% by weight or less.

2. The method according to claim 1, wherein the step (1) comprises providing a mixed solvent containing water and the organic solvent, and mixing the mixed solvent and the substance to be freeze-dried.

3. The method according to claim 1 or 2, wherein a weight percentage of the substance to be freeze-dried in a volume of the mixed solution containing water, the organic solvent, and the substance to be freeze-dried is 2 w/v% or more and 20 w/v% or less.

4. The method according to any one of claims 1 to 3, wherein the step (2) comprises a pre-freezing step.

5. The method according to any one of claims 1 to 4, wherein the step (2) comprises a drying step having a plurality of stages.

6. The method according to any one of claims 1 to 5, wherein a freezing point of the mixed solvent containing water and the organic solvent is -60°C or more.

7. The method according to any one of claims 1 to 6, wherein the organic solvent is t-butanol.

8. The method according to any one of claims 1 to 7, wherein the substance to be freeze-dried is a lipophilic peptide.

9. The method according to claim 8, wherein the lipophilic peptide has a cyclic structure.

10. The method according to claim 8 or 9, wherein a CLogP of the lipophilic peptide is 25 or less.

11. The method according to any one of claims 8 to 10, wherein a molecular weight of the lipophilic peptide is 5,000 or less.

12. The method according to any one of claims 8 to 11, wherein a number of amino acid residues of the lipophilic peptide is 5 or more and 30 or less.

13. The method according to any one of claims 8 to 12, wherein the lipophilic peptide contains a non-natural amino acid residue.

14. The method according to claim 13, wherein the non-natural amino acid residue is a non-natural N-substituted amino acid residue.

15. The method according to any one of claims 1 to 14, wherein a residual solvent concentration in a freeze-dried powder obtained by freeze-drying is 1.2% or less.

**INTERNATIONAL SEARCH REPORT**

International application No.
**PCT/JP2021/047164**

**A. CLASSIFICATION OF SUBJECT MATTER**

***A61K 9/19***(2006.01)i; ***A61K 38/13***(2006.01)i; ***F26B 5/06***(2006.01)i
FI: A61K9/19; A61K38/13; F26B5/06

According to International Patent Classification (IPC) or to both national classification and IPC

**B. FIELDS SEARCHED**

Minimum documentation searched (classification system followed by classification symbols)

A61K9/19; A61K38/13; F26B5/06

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | VAN DROOGE, D. J. et al. Incorporation of Lipophilic Drugs in Sugar Glasses by Lyophilization using a Mixture of Water and Tertiary Butyl Alcohol as Solvent. JOURNAL OF PHARMACEUTICAL SCIENCES. 2004, vol. 93, no. 3, pp. 713-725<br>abstract, section "Manufacture of solid dispersion through lyophilization", table 4 | 1-15 |
| A | JP 2019-511484 A (HEIDELBERG PHARMA RESEARCH GMBH) 25 April 2019 (2019-04-25)<br>paragraphs [0001], [0136] | 1-15 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 February 2022** | **08 March 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2021/047164**

| Patent document cited in search report | Publication date (day/month/year) | Patent family member(s) | Publication date (day/month/year) |
|---|---|---|---|
| JP 2019-511484 A | 25 April 2019 | EP 3222292 A1<br>paragraphs [0001], [0144] | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Biopolymers.,* 2000, vol. 55 (3), 227-250
- *Journal of Pharmaceutical Machinery and Engineering,* 2015, vol. 24 (2), 15, , 39
- *European Journal of Pharmaceutical Sciences,* 2002, vol. 15, 115-133
- *Journal of Pharmaceutical Sciences,* 2018, vol. 107, 2005-2012
- *Journal of Pharmaceutical Sciences,* 2018, vol. 107, 887-896
- *Journal of Pharmaceutical Sciences,* 2019, vol. 108, 399-415
- *ICHQ3C guideline; Q3C(R8) impurities: Guideline for Residual Solvents,* 25 March 2020
- *Journal of Pharmaceutical Sciences,* 2002, vol. 91, 1147-1155
- *Pharmaceutical Research,* December 2008, vol. 25 (12), 2799-2806
- *Acta Pharmaceutical Sinica,* 2007, vol. 42 (3), 314-317